# EUROPEAN PATENT SPECIFICATION

(11) **EP 3 256 212 B1**
(45) Date of publication and mention of the grant of the patent: **01.07.2020**
(21) Application number: 16747382.6
(22) Date of filing: 05.02.2016
(51) Int. Cl.: A61N 5/06

(54) **SYSTEMS FOR TARGETED UVB PHOTOTHERAPY FOR DERMATOLOGIC DISORDERS AND OTHER INDICATIONS**
SYSTEME ZUR GEZIELTEN UVB-LICHTTHERAPIE GEGEN DERMATOLOGISCHE ERKRANKUNGEN UND ANDERE INDIKATIONEN
SYSTÈMES POUR PHOTOTHÉRAPIE UVB CIBLÉE POUR TROUBLES DERMATOLOGIQUES ET AUTRES INDICATIONS

(30) Priority: 05.02.2015 US 201562112248 P; 28.07.2015 US 201562198070 P
(43) Date of publication of application: 20.12.2017
(73) Proprietor: BeneSol, Inc., Bainbridge Island, WA 98110 (US)
(72) Inventor: MOFFAT, William A., Bainbridge Island, WA 98110 (US)
(74) Representative: Walker, Ross Thomson
(86) International application number: PCT/US2016/016873
(87) International publication number: WO 2016/127120

(56) References cited:
- WO-A1-2015/061773
- DE-A1- 19 622 074
- US-A1- 2004 138 726
- US-A1- 2005 143 793
- US-A1- 2006 151 709
- US-A1- 2006 206 173
- US-A1- 2008 103 560
- US-A1- 2013 172 963
- US-A1- 2013 172 963

## Description

### TECHNICAL FIELD

The present technology relates to phototherapy, and more particularly to UVB phototherapy for treating dermatologic disorders and other indications.

### BACKGROUND

Some of the most common dermatologic disorders include psoriasis, atopic dermatitis (eczema) and vitiligo, affecting 3%, 2% and 1% of the general adult population, respectively. Psoriasis is a chronic relapsing/remitting auto-immune skin disease characterized by red, scaly patches and plaques that typically itch. The skin lesions seen in psoriasis may vary in severity from minor localized patches to complete body coverage. Atopic dermatitis, also known as eczema, is an inflammatory, relapsing, and non-contagious skin disorder. Both genetics and environmental allergen exposure can contribute to the development of this condition. People with eczema have itchy, red, dry, weeping, scaly skin patches that often span the entire body. Both Eczema and Psoriasis result from overactive immune response from various skin cells so the immune suppressing effect of UV light exposure has been suspected to be useful for these conditions since 1925.

There are several other dermatological disorders that are related to immune system dysfunction. Vitiligo is an autoimmune condition that causes non-uniform depigmentation of the skin when melanocytes (skin pigment cells) die or are unable to function. Chronic urticaria (commonly known as hives) is a skin rash notable for pale red, raised, itchy bumps that may cause burning or stinging sensation. It is well-recognized that 30-40% of chronic urticaria is autoimmune in nature. Lichen planus is a chronic, inflammatory, autoimmune disease that affects the skin with polygonal, flat-topped, purple papules and plaques that itch. Cutaneous T cell lymphoma is a type of cancer of the immune system caused by the mutation of T cells. Parapsoriasis is skin disorder that is considered an early stage of cutaneous T cell lymphoma and has symptoms that resemble psoriasis (red, scaly lesions). Pityriasis lichenoides is a skin condition characterized by groups of red, inflamed, and scaly papules that may persist for months, which is likely caused by an abnormal immune response to an antigenic trigger. Pityriasis rosea is an acute inflammatory disease of the skin thought to be association with cellular immunity and is characterized by scaly pink lesions on the torso, arms, and legs. Pruritus (itchy skin) is a common symptom in patients with end-stage renal disease that has a significant negative impact on quality of life.

Dermatologic disorders such as psoriasis and atopic dermatitis (eczema) have been treated with phototherapy. Because many dermatologic disorders are thought to be caused by a dysfunction of the dermal immune system, phototherapy efficacy on such conditions has been attributed to both the local and systemic immune system impact of ultraviolet light. Phototherapy is used by itself or in conjunction with topical calcitriol ointments, which are expected to provide more efficacious results. The skin endogenously produces calcitriol when exposed to specific wavelengths of UV light. However, the efficiency of immune system impact and cutaneous calcitriol production is highly wavelength dependent. Reference is made to document US2006/0206173 A1.

### BRIEF DESCRIPTION OF THE DRAWINGS

The invention and its preferred embodiments are defined in the appended set of claims and explained hereinafter. Many aspects of the present technology can be better understood with reference to the following drawings. The components in the drawings are not necessarily to scale. Instead, emphasis is placed on illustrating clearly the principles of the present technology.
Figure 1 is a graph illustrating phototherapy emission spectra for various types of UV emitting devices.
Figure 2 is a graph illustrating the psoriasis phototherapy action spectrum.
Figure 3 is a graph illustrating the contact hypersensitivity action spectrum.
Figure 4 is a graph illustrating the cis-urocanic acid action spectrum.
Figure 5 is a graph illustrating in vivo thymine dimer action spectra.
Figure 6 is a graph illustrating the in vivo tumor necrosis factor alpha action spectrum.
Figure 7 is a graph illustrating the psoriasis phototherapy action spectrum and immune response action spectra.
Figure 8 is a graph illustrating an immune response phototherapy action spectrum configured in accordance with embodiments of the present technology.
Figure 9 is a graph illustrating the pre-vitamin D3 action spectrum.
Figure 10 is a graph illustrating the pre-vitamin D3 action spectrum and the vitamin D3 action spectrum.
Figure 11 is a graph illustrating phototherapy emission spectra for various types of UV emitting devices and the vitamin D3 action spectrum.
Figure 12 is a graph illustrating a calcitriol action spectrum.
Figure 13 is a graph illustrating the erythema action spectrum.
Figure 14 is a graph illustrating UVB phototherapy emission spectra, the immune response phototherapy action spectrum of Figure 8, and the erythema action spectrum of Figure 13.
Figure 15 is a graph illustrating the vitamin D₃/calcitriol action spectrum and the immune response phototherapy action spectrum of Figure 8.
Figure 16 is a graph illustrating a combined phototherapy action spectrum configured in accordance with embodiments of the present technology.
Figure 17 is a table illustrating the relationship between skin type, Minimum Erythema Dose (MED), Standard Erythema Dose (SED), and Erythemal Effective Radiant Exposure (EERE).
Figures 18-32 are dosage tables illustrating skin type dependent parameters of phototherapy sessions for focused UV phototherapy devices having differing spectral irradiances.
Figure 33 is an isometric view of a high-energy phototherapeutic apparatus or system for focused UV radiation configured in accordance with an embodiment of the present technology.
Figure 34 is an isometric view of a low-energy phototherapeutic apparatus or system for focused UV radiation configured in accordance with another embodiment of the present technology.
Figure 35 is a block diagram illustrating an overview of devices on which some implementations of the present technology may operate.
Figure 36 is a block diagram illustrating an overview of an environment in which some implementations of the present technology may operate.

### DETAILED DESCRIPTION

The present technology is directed to phototherapy devices, systems, and methods that provide specific wavelength-focused UV and are expected to increase or maximize both immune system impact and calcitriol production, as well as reduce total UV exposure. Such systems and methods can improve the efficacy of combination phototherapy without the need for additional calcitriol ointment application. Although many of the embodiments are described below with respect to systems, devices, and methods for treating psoriasis and promoting vitamin D production in the skin, other applications (e.g., phototherapeutic treatment of other dermatological disorders and/or autoimmune diseases) and other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described below with reference to Figures.

### I. Phototherapy Treatment

Phototherapy is one of the most efficacious and popular treatment options for all of these moderate to severe dermatologic disorders. Phototherapy is considered a first-line remedy for extensive psoriasis, with treatment usually consisting of 3 exposures per week for at least 3 months. There are several local and systemic immune-modulating biological mechanisms that contribute to the efficacy of this treatment. Phototherapy has been shown to alter the helper T cell-derived cytokine profile, both within the psoriatic lesions and systemically, to suppress the dysfunctional overactive immune response. Studies have shown that apoptosis of keratinocytes and depletion of T lymphocytes leads to the local immune suppression responsible for lesion clearing. Phototherapy has been shown to suppress and reduce the number of langerhans cells (antigen-presenting immune cells of the skin) in response to keratinocyte apoptosis causing local immune suppression. Full body UVA and/or UVB irradiation will cause production of cis-urocanic acid and DNA pyrimidine dimers, which can lead to systemic immune suppression and is expected to be an effective tool for restoring immune function. Ultraviolet phototherapy has been used to treat various dermatologic disorders including psoriasis, atopic dermatitis, vitiligo, chronic urticaria, lichen planus, cutaneous T cell lymphoma, pityriasis lichenoides, parapsoriasis, pityriasis rosea, pruritus, seborrheic dermatitis, and actinic prurigo.

### II. Phototherapy Sources

Ultraviolet exposure at various wavelengths has been found to have a beneficial impact on dermatological disorders. Sun exposure itself has been shown to produce both local and systemic immune suppression that benefits these conditions. Many phototherapy devices provide controlled delivery of ultraviolet radiation using various wavelength combinations, including: broadband UVB (280 nm-320 nm); narrowband UVB (311 nm-313 nm); excimer laser (308 nm); UVA (340 nm-400 nm); and UVA with psoralen (PUVA). Each technology provides a different spectrum of UV light, but all work on the same principle of immune suppression. Broadband UVB (BB-UVB) is expected to elicit both local and systemic immune suppression. Narrow-Band UVB (NB-UVB) is also expected to elicit local and systemic immune suppression. PUVA treatment uses psoralen as a photosensitizer and subsequent UVA exposure to cause local and systemic immune suppression.

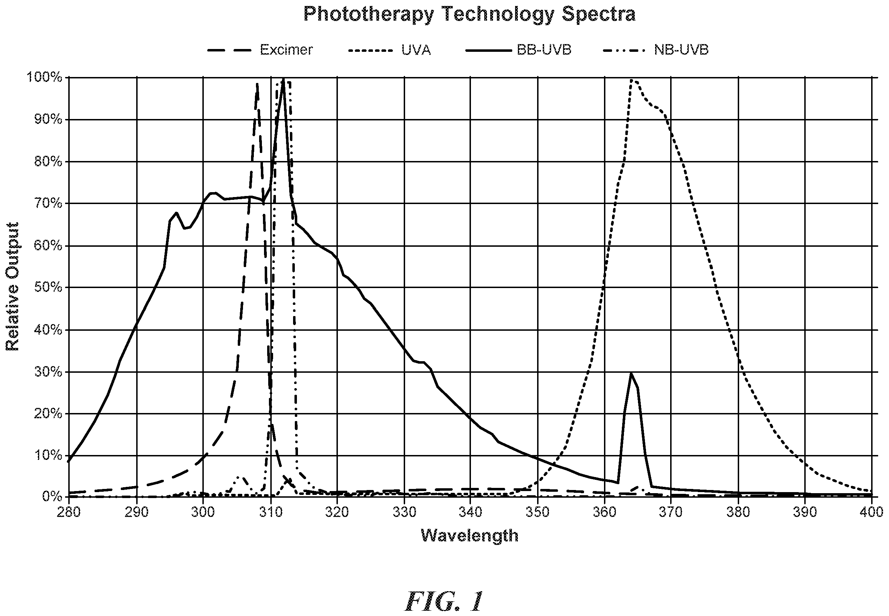

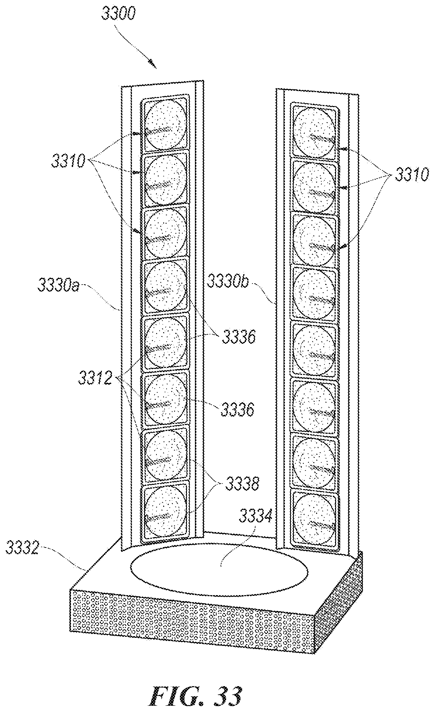

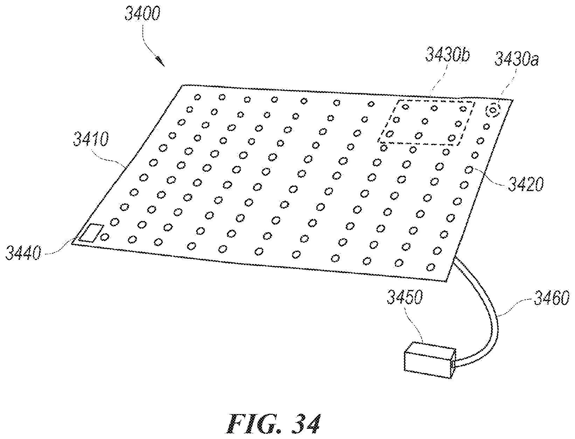

Phototherapy using UVA, NB-UVB, and excimer laser has also been used to effectively treat vitiligo, likely due to local immune suppression. Studies have shown that NB-UVB is more effective than UVA and excimer laser is more effective than NB-UVB at stimulating repigmentation of melanocytes affected by vitiligo, demonstrating a preference toward shorter wavelength UVB. As discussed in further detail below, the preference for UVB emissions is consistent with several immune suppression action spectra. The spectral analysis of phototherapy technologies for excimer lamps, UVA devices, broad-band UVB devices, and narrow-band UVB devices is shown in Figure 1. As described in further detail below with reference to Figures 33 and 34, these phototherapeutic radiation sources can be incorporated into phototherapeutic devices and systems.

### III. Immune Response Action Spectra

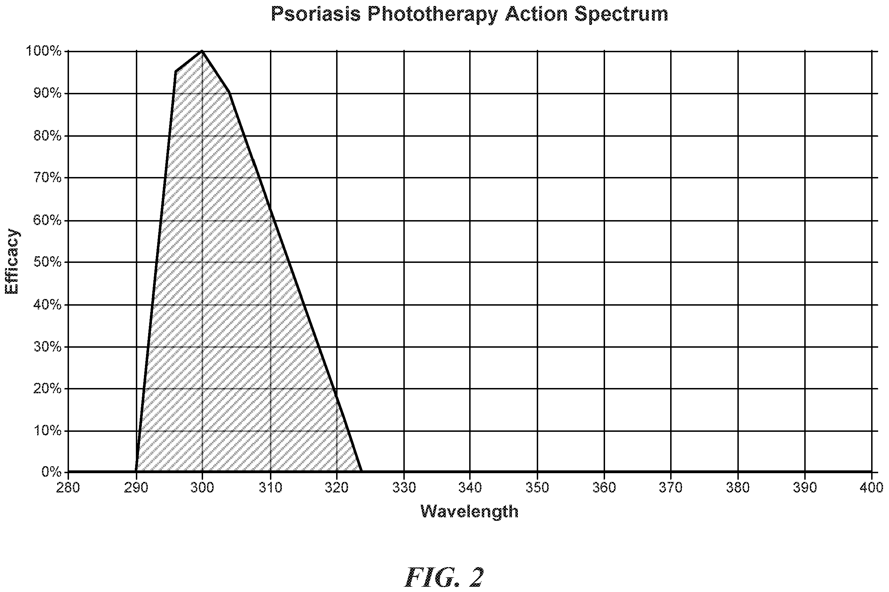

Not all wavelengths of ultraviolet light have the same effectiveness for treating dermatologic disorders. The relative wavelength effectiveness (i.e., action spectrum) has been determined for a few indicators of phototherapy efficacy. For example, the in vivo action spectrum for phototherapy of psoriasis is shown in Figure 2.

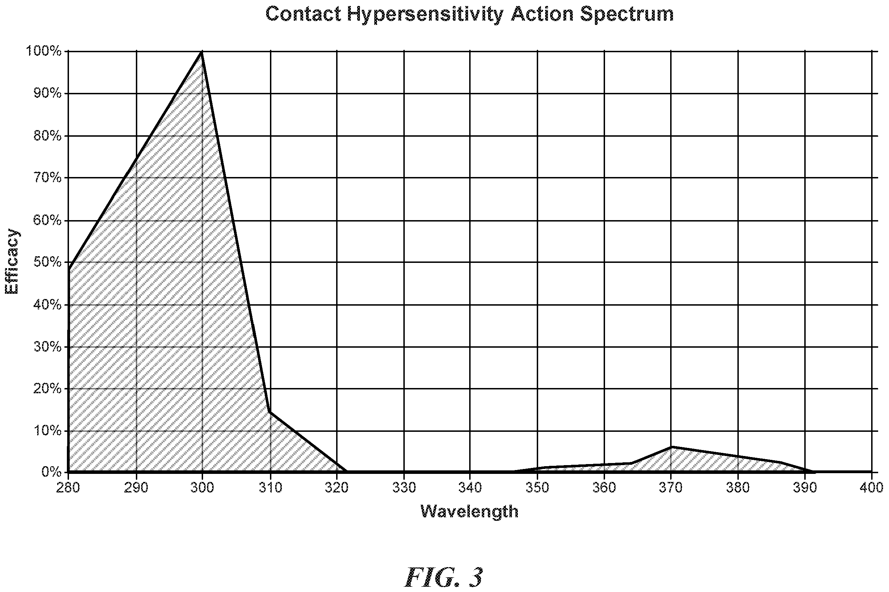

The action spectra for various measures of biological mechanisms thought to be responsible for the immune response have also been shown to be effective for treating dermatologic disorders. The in vivo action spectrum for the induction of systemic suppression of contact hypersensitivity (a measure systemic immune alteration) is shown in Figure 3.

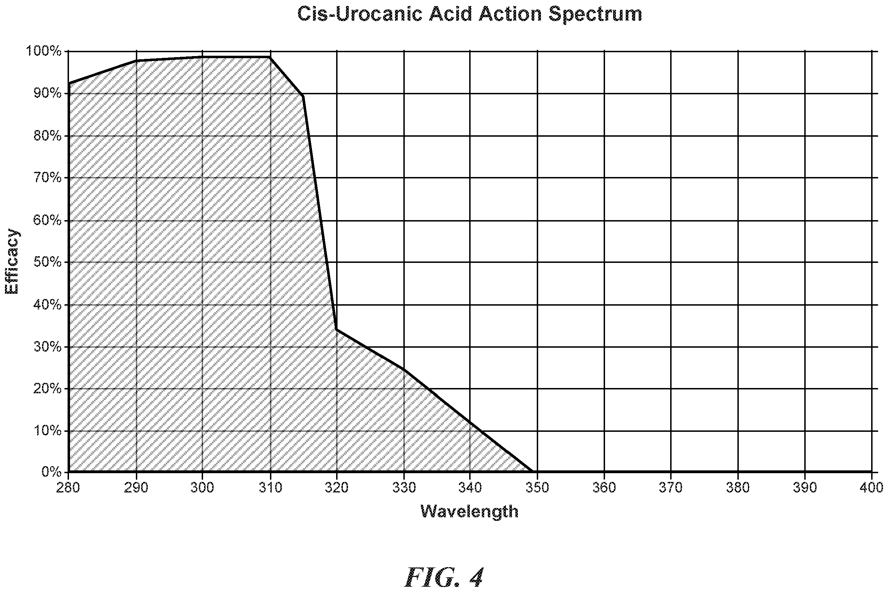

Cis-urocanic acid is a sunlight-induced systemic immunosuppressive factor that also is also expected to have an impact on dermatological skin disorders. An action spectrum for cis-urocanic acid production in human skin is shown in Figure 4 and illustrates a peak in the UVB spectral region of 290 nm-310 nm.

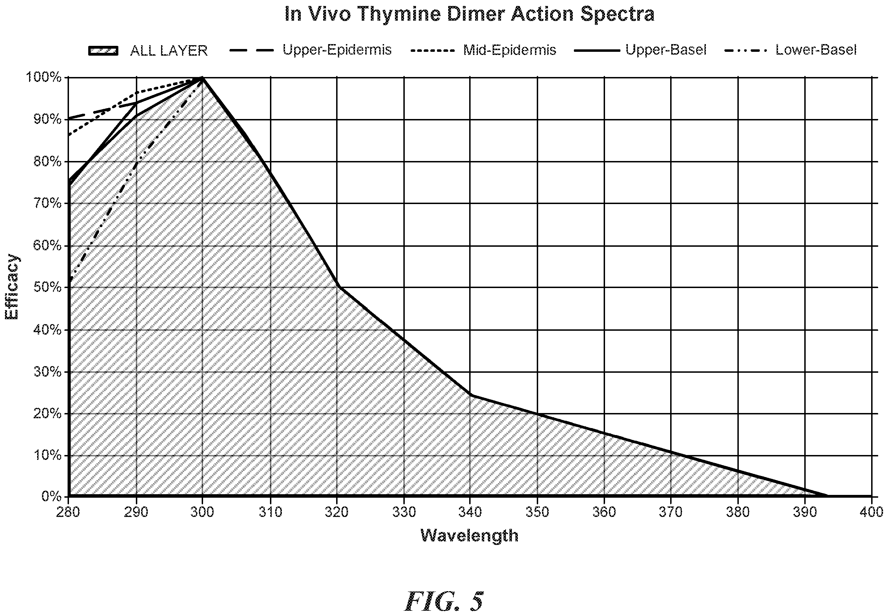

Ultraviolet light causes direct DNA damage in the form of pyrimidine dimers and (6-4) photoproducts, which induce apoptosis of keratinocytes. This activates antioxidant DNA repair enzymes, as well as local and systemic immune suppression that can be effective for psoriasis. The in vitro action spectrum for the formation of thymine dimers and (6-4) photoproducts in DNA shows a peak near 260 nm. However, the vivo action spectrum for epidermal thymine dimer formation shows a peak at 300 nm for all skin layers. The longer peak wavelength is thought to be caused by the significant reduction in epidermis transmission of UV wavelengths shorter than 300 nm. Figure 5 illustrates an average in vivo thymine dimer action spectrum based on dimer formation for all skin layers tested in the study.

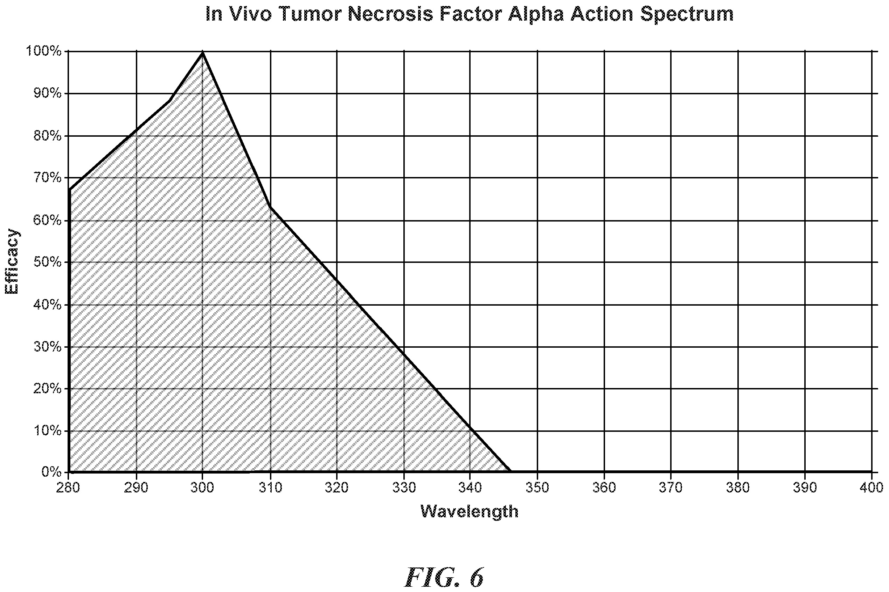

Tumor necrosis factor alpha has been found to be an important initiator of the cytokine profile change seen in the skin after UV exposure that favors anti-inflammatory response. An action spectrum for in vivo production of tumor necrosis factor alpha is shown in Figure 6.

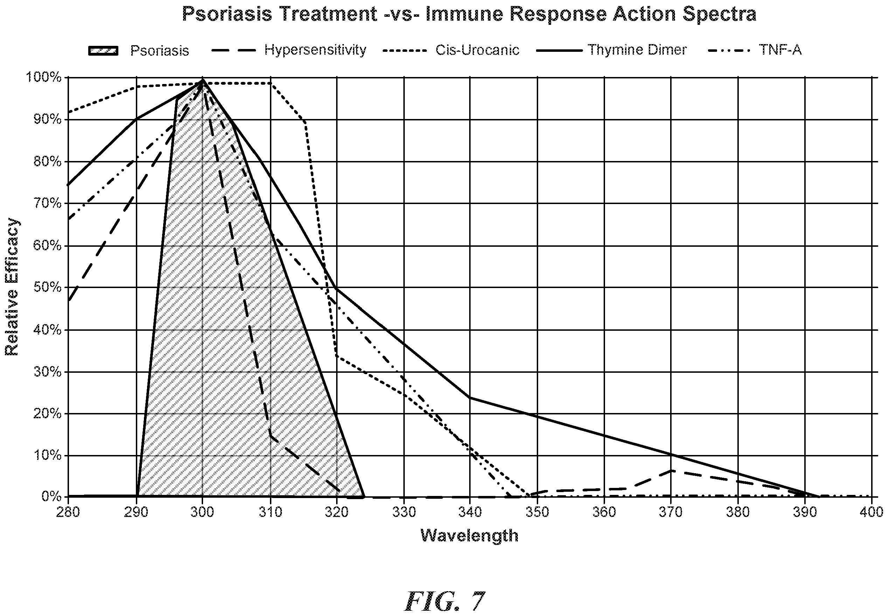

As shown in Figure 7, the action spectrum for phototherapy specific to psoriasis is very similar to the other action spectra for biological mechanisms responsible for phototherapy efficacy in that all the action spectra have a peak at or near 300 nm.

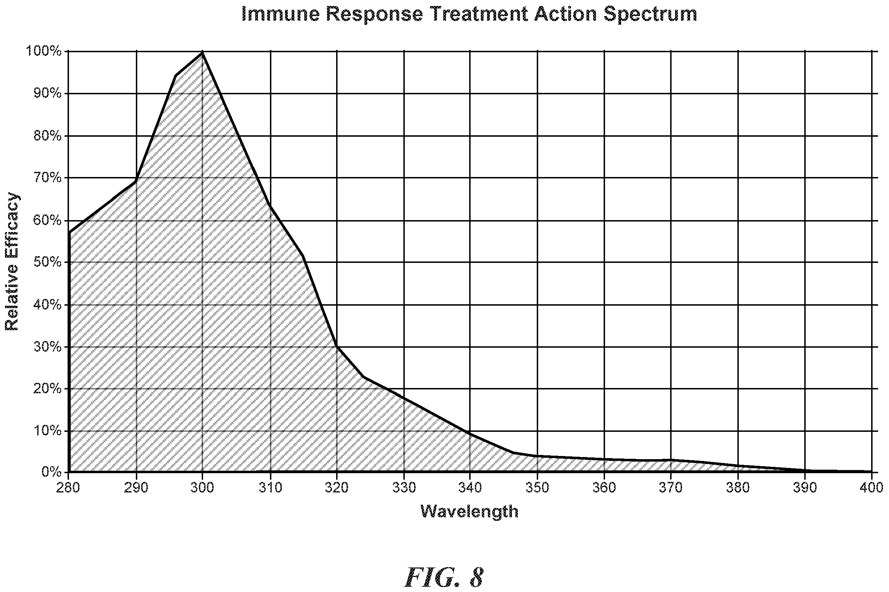

To simplify the expression of psoriasis treatment and multiple established action spectra for systemic and local immune response, Figure 8 shows a single action spectrum for immune response treatment of dermatologic disorders. This single action spectrum represents the average efficacy for psoriasis treatment, suppression of contact hypersensitivity, cis-urocanic acid production, all skin layer thymine dimer formation and tumor necrosis factor alpha production at each wavelength of irradiance. The resultant combination action spectrum demonstrates the wavelengths of light that are most effective to elicit local and systemic immune response needed to treat immune-mediated dermatologic disorders with minimum total irradiance per phototherapy treatment.

### Vitamin D₃

In recent years vitamin D₃ has been identified as the major regulator of cathelicidin expression. Cathelicidin is an antimicrobial peptide found in the skin that is impacts both innate and adaptive immunity. Dysfunctional cathelicidin expression has been observed in dermatological disorders such as atopic dermatitis and psoriasis. Additionally, 25-hydroxyvitamin D₃ insufficiency has been correlated with certain dermatological disorders, such as psoriasis, atopic dermatitis, and vitiligo. Correcting 25-hydroxyvitamin D₃ deficiency in patients with dermatological disorders is expected be a contributing factor in the efficacy of existing UVB phototherapy devices. For example, a study has shown that high dose vitamin D was an effective treatment for psoriasis and vitiligo.

### Cutaneous Vitamin D₃ Production

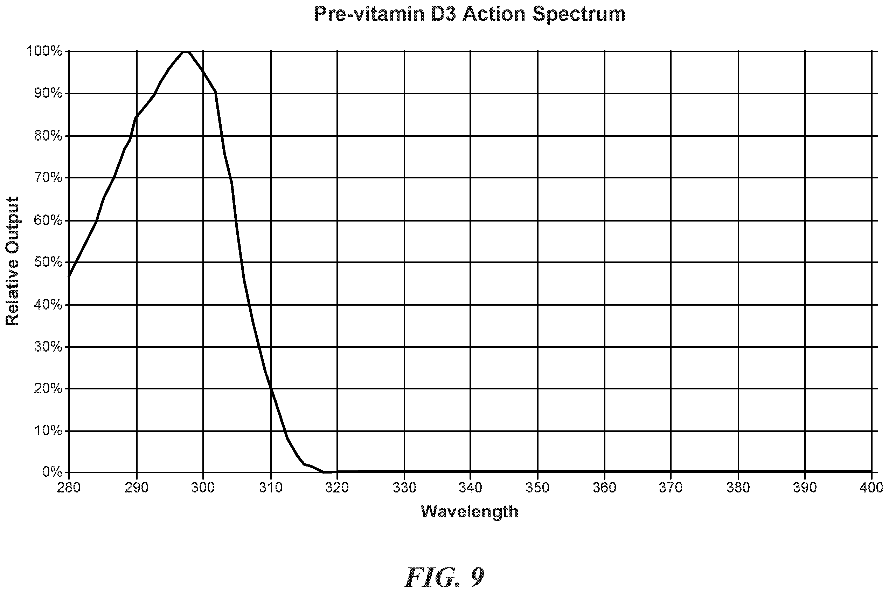

When human skin is exposed to UVB light (280 nm-315 nm) it converts 7-dehydrocholesterol (7-DHC) to pre-vitamin D₃ (as well as two other biologically inert photoproducts that regulate production). Pre-vitamin D₃ is converted to vitamin D₃ in the skin and then transferred to the blood stream over the course of several days. These internal controls result in a deliberately regulated, slow and steady trickle of vitamin D₃ to the liver, lasting more than two weeks. After arriving in the liver, vitamin D₃ requires two metabolic conversions (25-hydroxylation in the liver and then 1alpha-hydroxylation in the kidney) to become the active pro-steroid hormone calcitriol. Figure 9 illustrates monochromatic UV action spectrum for the conversion of 7-DHC to pre-vitamin D₃ in human skin. As shown in the graph, the peak synthesis occurs at 297-298 nm. The same data was further defined and extended by the International Commission on Illumination ("CIE").

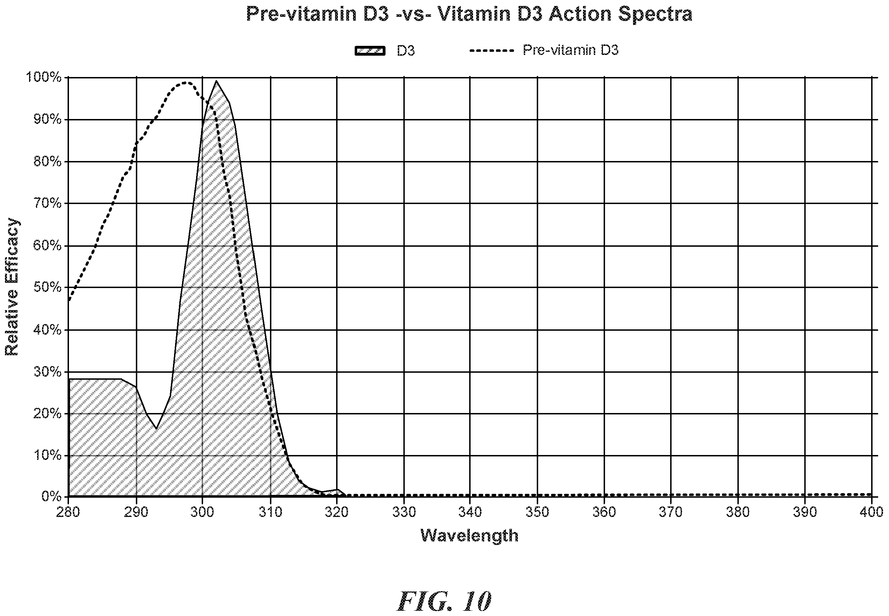

A vitamin D₃ action spectrum was constructed using human skin equivalent exposed to therapeutic doses of UV, showing a peak at 302 nm. The comparison between the pre-vitamin D₃ and vitamin D₃ action spectra is shown in Figure 10.

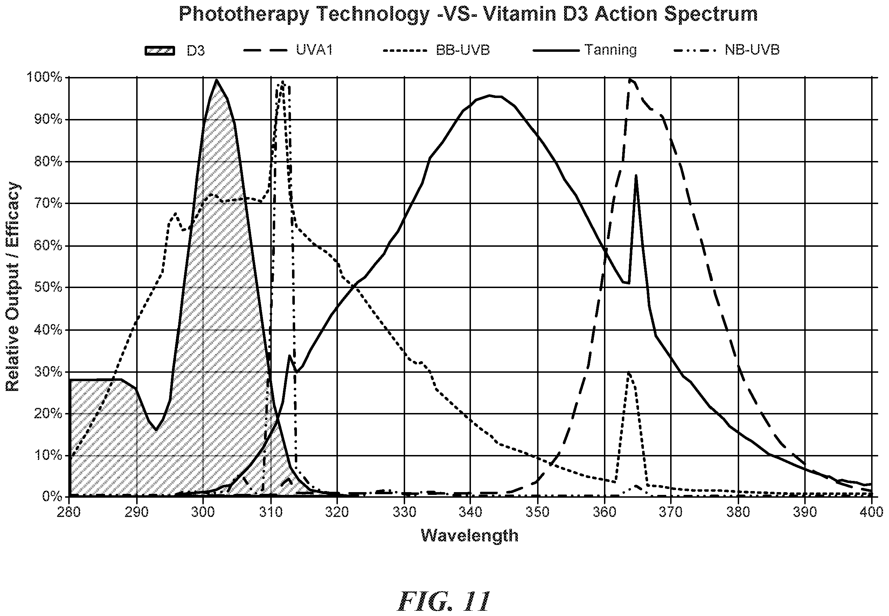

In Figure 11, the comparison between the vitamin D₃ action spectrum and spectral analysis of four common forms of phototherapy (BB-UVB, NB-UVB, UVA, and tanning) indicates that each phototherapy technology has a different propensity to produce vitamin D₃. Indeed, only phototherapy using UVB can produce significant alteration to serum vitamin D concentration as the UVA spectrum is outside the pre-vitamin D₃ action spectrum. Furthermore, because a larger amount of energy in BB-UVB is within the most effective range of the pre-vitamin D₃ action spectrum, BB-UVB produces more vitamin D than NB-UVB. However, none of the current phototherapy technologies optimize vitamin D₃ production.

### Calcitriol

The active form of vitamin D is known as calcitriol. The epidermis possesses receptors for calcitriol which inhibit the proliferation of cultured keratinocytes and induces them to terminally differentiate. Vitamin D receptor research confirms an association of calcitriol with susceptibility to psoriasis. Both oral and topical administration of calcitriol have been used as treatments for psoriasis and vitiligo, with topical solutions typically being more efficacious and safer than oral options, which carry a risk of hypercalcemia. Topical synthetic calcitriol is often used in combination with phototherapy because it is expected to increase overall efficacy with fewer UV treatments and less total UV exposure. However, topical calcitriol is degraded by UV light, so efficacy is significantly reduced when applied before phototherapy treatment.

### Cutaneous Calcitriol Production

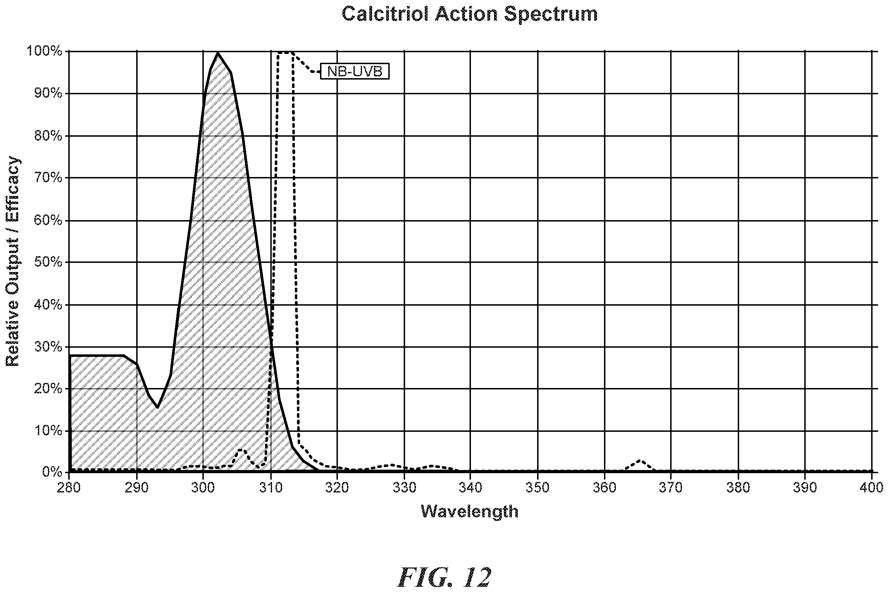

Vitamin D from cutaneous synthesis or dietary intake is sequentially converted in the liver to 25-hydroxyvitamin D₃ and then in the kidneys to calcitriol. However, it has been shown that in addition to this internal process, calcitriol is produced directly in human skin exposed to UVB. Researchers suggest that cutaneous synthesis of calcitriol could be a contributing factor to the effectiveness of current UVB-based phototherapy. Calcitriol photoproduction in the skin is highly sensitive to wavelength, similar to vitamin D₃, with studies demonstrating maximized formation between 300 nm and 305 nm. In fact, the amount of vitamin D₃ photoproduction in the skin directly determines the amount of subsequent calcitriol conversion in the skin. The same study that constructed the vitamin D₃ action spectrum also determined that the action spectrum for subsequent calcitriol production is identical (Figure 12).

A narrowband TL-01 lamp made by Philips of Andover, MA, which is a commonly used UVB source for phototherapy, has maximum spectral irradiance at around 311 nm. As shown in Figure 12, the spectral curve of the TL-01 NB-UVB lamp does not overlap much with the calcitriol action spectrum. Thus, while the TL-01 lamp can produce a small amount of calcitriol, UVB energy at 300 nm (+/- 2.5 nm) is significantly more effective at producing calcitriol (e.g., 38 times more effective).

### IV. Erythema & MED

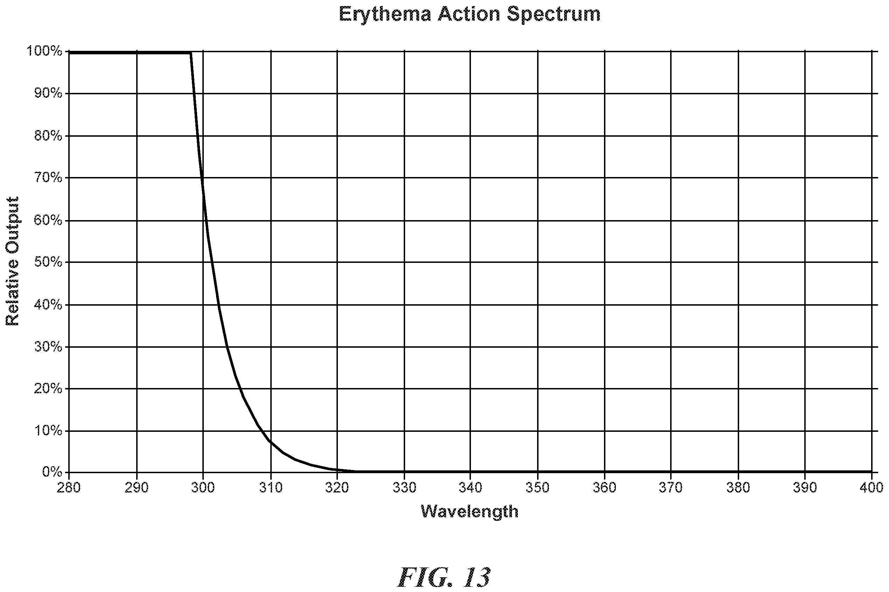

Erythema is redness of the skin caused by increased blood flow which occurs with skin injury, infection, or inflammation. Erythema caused by UV exposure is commonly referred to as sunburn. The Erythema Reference Action Spectrum and Standard Erythema Dose (SED), internationally recognized standard published by the CIE (ISO 17166:1999), is used to determine erythema response to individual wavelengths from 250 nm-400 nm. The CIE action spectrum for erythema is used as a weighting factor for spectral irradiance output from a UV source used for phototherapy treatment. As shown in Figure 13, the erythema action spectrum has a constant maximum from 250 nm-298 nm, falls off rapidly between 298 nm and 325 nm, then declines slowly and steadily thereafter.

Standardized ultraviolet doses used in phototherapy treatment are based on the individual patient's Minimal Erythemal Dose (MED) for a given light source. The amount of erythemally weighted UV radiation necessary to produce a slight pink coloration of the skin within 24 hours in called 1 MED. The erythema response of skin to UV radiation is correlated to constitutional skin color which is determined by melanin content. Individuals with darker skin color have more melanin absorbing UVB photons. Therefore dark skin requires more erythemally weighted UV than light skin to achieve a standard MED dosage. Historically, phototherapy applications have used the Fitzpatrick Skin Type classification system to place the constitutive skin color of a patient into one of six classes. According to the Fitzpatrick system, skin type 1 has the lightest skin color (lowest melanin content) and skin type 6 has the darkest skin color (highest melanin content).

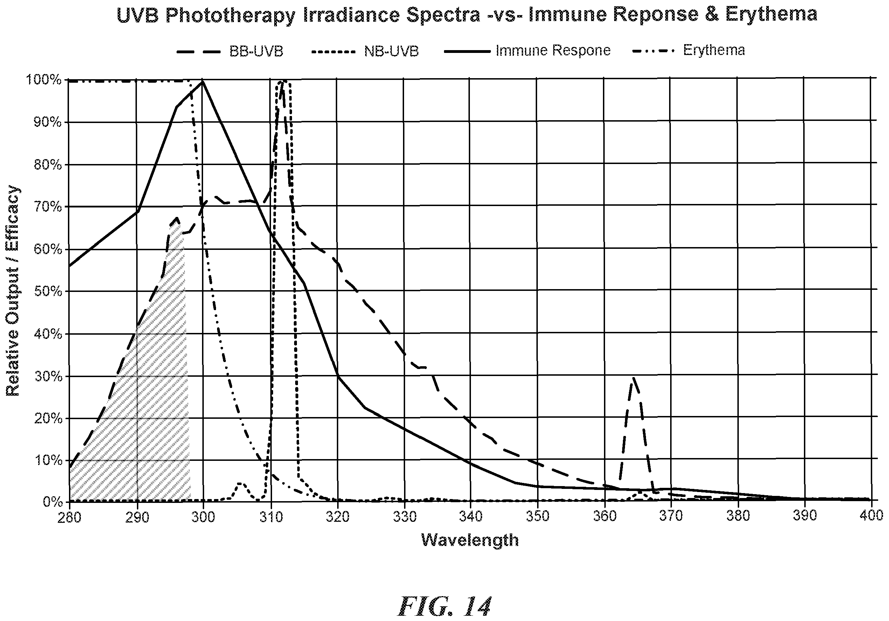

The relationship between erythema and immune response at each wavelength can be important for determining the most effective UV source for dermatologic treatment. Specifically, the spectral irradiance of a UV source should deliver energy in a range of wavelengths where the ratio between erythema and immune response is less than 1. Therefore, delivering UV energy with wavelengths shorter than 298 nm would provide progressively diminished therapeutic benefit because the wavelength is reduced to levels below maximum immune response (e.g., approximately 300 nm as shown in Figure 8), while erythema remains at a constant maximum. Figure 14 indicates that most of the spectral energy from narrowband UVB (NB-UVB) has an erythema/immune response ratio less than 1, while broadband UVB (BB-UVB) contains significant energy that contributes to erythema more than immune response (i.e., see shaded area of Figure 14). Thus, Figure 14 indicates that more total UV energy with greater immune response can be delivered per standardized MED treatment using NB-UVB rather than BB-UVB. Consequently, it has been found that NB-UVB is more effective at treating psoriasis than BB-UVB.

### V. Combination Action Spectrum

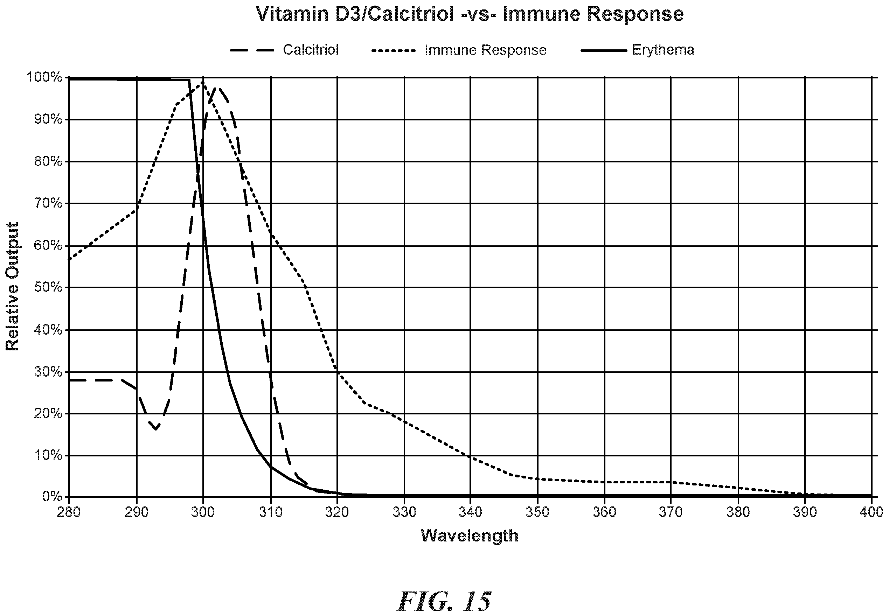

The erythema, pre-vitamin D₃, vitamin D₃, calcitriol, psoriasis clearance, and several immune response action spectra have been defined and, as shown in the Figures, are very similar to each other. In Figure 15, the action spectrum for vitamin D₃/calcitriol photoproduction is shown in comparison to the erythema action spectrum and an action spectrum constructed from multiple immune response spectra (i.e., the immune response treatment action spectrum of Figure 8).

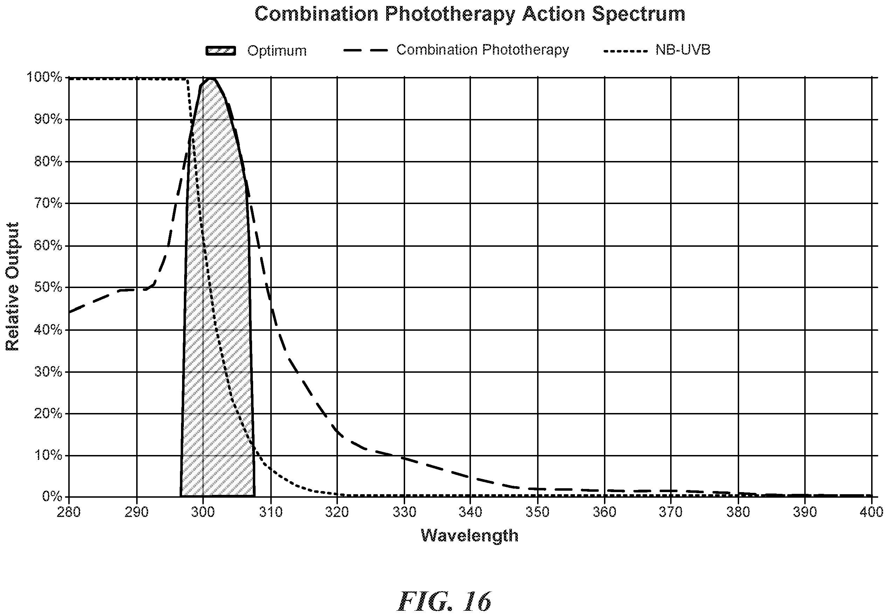

Figure 16 illustrates a "combination phototherapy action spectrum" that includes the average of the vitamin D3/calcitriol action spectrum of Figures 10 and 12 and the immune response action spectrum of Figure 8. This combination action spectrum expresses the maximum efficacy for both immune response and vitamin D₃/calcitriol production in the skin. Using a phototherapy device (for treatment of a dermatologic disorder) that isolates and delivers to the skin a UV spectrum maximizing calcitriol production and immune response under the action spectrum of Figure 16 is expected to provide the increased efficacy of combination therapy without the need for additional oral or topical calcitriol administration. As shown in Figure 16, the optimal wavelength range for maximum phototherapy efficacy is between 298 nm and 307 nm, with minimal UV energy at wavelengths shorter than 298 nm or longer than 307 nm. Accordingly, a phototherapy device, such as those described in further detail below with reference to Figures 33 and 34, that emits more than 75% of total UV output within the wavelength range 298 nm to 307 nm is expected to be most effective and safe for the treatment of dermatologic disorders.

### VI. Treatment Dosage

Phototherapy dosage can be described as the intensity (or irradiance) of a light source multiplied by time of exposure to that light source (Dose = Intensity x Time). Therefore, a desired dosage may be achieved by increasing or decreasing the intensity of the radiation source and/or exposure time. Dosage may be expressed in millijoules per centimeter squared (mJ/cm²) when intensity (or irradiance) is expressed in milliwatts per centimeter squared (mW/cm²), and time can be expressed in seconds. As explained in further detail below, for phototherapy applications, several additional factors can influence the calculation of intensity and dosage for a particular radiation source and configuration of that source relative to the patient.

### Dosage and Selected Embodiments of Phototherapy Sources and Systems

Phototherapy can be delivered to the skin with systems that provide a substantially uniform distribution of energy to the treatment area of the skin, and the uniformity with which the phototherapy is applied can affect the dosage level delivered during a phototherapy session. More specifically, the dosage delivered to the entire treatment area is generally limited by the largest dosage level applied to any one area of the skin. For example, if a treatment area is 100 cm² and the phototherapy system used to deliver phototherapy to the treatment area has a non-uniform energy distribution that exposes 10 cm² of the treatment area to twice the intensity as the intensity applied to the other 90 cm², the dosage of the entire treatment area is limited by the maximum dosage that can be applied to the 10 cm² treatment area. This results in a large portion of the treatment area being exposed to half of the maximum or desired dosage. Accordingly, phototherapy systems that emit radiation with greater uniformity are expected to enhance treatment efficacy.

Various mechanisms can be used to emit and apply the irradiance of a light source or system of light sources to the skin with relative uniformity. Selected embodiments of phototherapeutic systems with low- and high-energy radiation sources are described in further detail below with reference to Figures 33 and 34. In certain embodiments, a phototherapy device includes one or more low-energy radiation sources (e.g., 3 Watts or less) that can be positioned in close proximity to the treatment area on the patient (e.g., 3 cm or less). This allows the phototherapy to be delivered to selective and scalable treatment areas. In other embodiments, the phototherapy device includes one or more high-energy radiation sources (e.g., 25 Watts or greater) that are spaced apart from the treatment area on the patient by a distance large enough (e.g., 10 cm or more) to allow distribution of the emitted energy from the radiation sources. For example, the radiation sources may have an emission pattern that has an uneven distribution of intensity at a position close to the radiation source (e.g., a higher intensity at the center of the emission pattern), but that distributes light outwardly such that the radiation source provides a substantially uniform distribution of radiation intensity when spaced further from the radiation source. In this embodiment, the phototherapy can be applied over a large treatment area (e.g., 100 cm² or greater).

The low-energy phototherapy system can include one or more small, radiation sources with relatively monochromatic wavelength emissions. These radiation sources can be configured such that they do not require a separate filtering method (e.g., a coating) and may be assembled in tightly-packed arrays. For example, the radiation source can be a light emitting diode (LED). In a phototherapy system using LEDs as the radiation source, the LEDs can be configured to emit radiation at a specific wavelength target with most of the optical energy emitted within a small bandwidth (e.g., a 10 nm bandwidth) suitable for phototherapeutic treatment of dermatological disorders, vitamin D phototherapy, autoimmune disorders, and/or other indications. For example, the wavelengths of the LEDs can be selected using the methods described above with respect to Figures 1-16. In certain embodiments, the LEDs can emit wavelengths between 298 nm and 307 nm. In other embodiments, the LEDs can have one or more different wavelengths, such as wavelengths ranging from 295 nm to 310 nm. The individual LEDs can also include one or more lenses or other features that spread the emitted light at least substantially evenly across a surface area. A larger lens can be used in addition or as an alternative to the individual LED lenses, and placed over more than one LED to enhance the uniformity of emissions across several LEDs. In various embodiments, the LEDs of the phototherapy system are arranged in tightly packed arrays, such as arrays of 50 or more LEDs. The intensity of the LED array can be selected by adjusting various parameters of the array and associated components. For example, the intensity of the LED array can be increased by increasing the input energy delivered to the LEDs (e.g., by changing the power source or controls thereon), increasing the quantity of LEDs per unit area, decreasing the distance between the LEDs and the treatment area on the patient (e.g., 0-3 cm), decreasing the degree of light spreading of the lens(es) on the LEDs, and/or changing other features of the LED array that impact the radiation intensity. Conversely, the intensity of the LED array can be decreased by decreasing the level of energy delivered to the LEDs, decreasing the quantity of LEDs per unit area, increasing the distance between the LEDs and the treatment area on the patient, increasing the degree of light spreading of the lens(es) on the LEDs, and/or changing other features of the LED array that impact the radiation intensity.

The LED-based phototherapy system can provide an at least substantially uniform distribution of irradiation intensity by taking into account various features of the system, such as the distance between the LEDs and the treatment site on the patient, the spacing of the LEDs with respect to each other, and/or the shape of the lenses on the individual LEDs. For example, the LED array can be arranged such that at least a major portion of emission patters of the individual LEDs do not overlap each other such that irradiation from one LED of the array does not overlap the irradiation of another LED. The lenses on the individual LEDs can be used to expand or contract the LED emissions of the individual LEDs such that they do not overlap each other. In certain embodiments, LEDs are spaced apart by a distance that avoids overlapping LED emissions, but also leaves some portions of the treatment area (e.g., the area opposed to the area of the LED array or the area within emission area of the LED array) unexposed from the LED emissions. For example, the LEDs may be spaced apart by a distance such that 20% of the treatment area is not exposed to the LED emissions while the remaining 80% of the treatment area is exposed to a substantially uniform level of intensity from the LEDs. In other embodiments, the LEDs are spaced apart in such a manner that 30%, 40%, or 50% of the treatment area of the patient is unexposed, while the corresponding 70%, 60%, or 50% of the treatment area is exposed to a substantially uniform level of intensity.

When the LED-based phototherapy system is configured to provide an at least substantially uniform irradiation intensity, it is important that the LED array remain at a constant distance from the treatment area during the phototherapy session to maintain the uniform exposure to the phototherapy source. Accordingly, in certain embodiments the phototherapy device is designed to come in direct contact with the treatment area (e.g., the radiation source is placed on the patient's skin). For example, the phototherapy device can include a sensor that indicates when the device is appropriately placed on the skin to confirm direct skin contact before and/or during operation of the device during a phototherapy session. The phototherapy device can include a strap, an adhesive, and/or another type of fastener that allows the LED array to attach directly to the treatment area. In these embodiments, the constant distance from the skin surface to the radiation source is maintained by the device design itself, rather than being subject to movement of the patient or operator discretion.

Low-energy phototherapy devices, such as the LED-based device described above, can be a wearable device that can be attached to the patient or positioned immediately adjacent to the patient's skin. The wearable phototherapy device can include a radiation source (e.g., an LED array) affixed to a substrate, such as a flexible or non-flexible sheet or fabric that can carry the radiation source. The wearable phototherapy device can take the form of a pad or mat on which the patient can lay, sit, or stand, a patch that can be adhered to a patient's skin, panel incorporated into an article of clothing or other wearable item, a blanket, a cuff, a cap, a shirt, a jacket, pants (e.g., leggings), a sock, a glove, a vest, a cape, a watch, a wand, a paddle, a comb, and/or other suitable items that can be applied directly to the patient's skin. The wearable phototherapy device can be constructed to provide a substantially uniform and constant level of radiation intensity across the portion of the device including the radiation sources. In various embodiments, the wearable phototherapy device can also allow for adjustments in the dosage by altering input energy through system controls and/or time of exposure.

High-energy phototherapy systems can include one or more radiation sources that emit a large amount of energy in a selected UVB range (e.g., 298 nm to 307 nm) and a filtration mechanism that blocks unwanted wavelengths outside of the selected range. The radiation source can include one or more mercury arc lamps, pulse and flash xenon lamps, fluorescent lamps, metal halide lamps, and/or other suitable radiation sources for phototherapy. The phototherapy apparatus can include a plurality of radiation sources, such as 5 lamps, 10 lamps, 20 lamps, 30 lamps, 40 lamps, 50 lamps, or more depending on the type of lamp, the desired size of the treatment area, the desired intensity, and the desired phototherapy time. In certain embodiments, the radiation source itself can include filtration mechanisms. In other embodiments, the phototherapy system includes additional filtering features separate from the radiation source to emit the desired wavelength range. The filtration mechanism can include absorption filters and/or interference filters. The high-energy phototherapy system can provide an at least substantially uniform distribution of irradiation intensity by taking into account various features of the system, such as the distance between the radiation sources (e.g., no overlapping emission patterns), the shape of any lenses on the radiation sources, and the distance the patient must be positioned away from the radiation sources to receive substantially uniform irradiation distribution. In addition, the output of the phototherapy system may be adjusted by changing the energy input, the number of lamps, lens specifications, and/or filtration parameters.

### Intensity (or Irradiance)

The intensity of a radiation source can be measured as the absolute milliwatts per centimeter squared (mW/cm²) measured at a given distance from the source. As the distance between the source and measurement position increases, the intensity of the measurement will decrease. In high-energy phototherapy devices, the intensity of a phototherapy device is assumed to be measured at the position of the patient relative to the radiation source. If the distance from the radiation source to the patient greatly between patients, between phototherapy sessions, or along the body of a single patient, the uniformity and intensity of the irradiance becomes too varied for consistent dosages of phototherapy applications. Accordingly, in various embodiments, phototherapeutic devices can be configured such that the distance of the patient from the radiation source is no less than 10 cm and no greater than 100 cm. Within this range, a standard position for the patient can be determined for a phototherapy device configuration such that the variation of the patient position is no greater than about 30 cm. In low-energy devices, the radiation source is assumed to be directly in contact with the patient's skin, or at no greater distance from the treatment site than 3 cm.

Intensity for a radiation source in phototherapy applications uses "The Erythema Reference Action Spectrum" (ISO 17166:1999) as a weighting factor for spectral irradiance output measurement. The absolute measured intensity (mW/cm²) for each wavelength is multiplied by the weighting factor for that wavelength to determine the erythemally weighted irradiance. The sum of all erythemally weighted irradiance for each individual wavelength equals the total erythemally weighted irradiance (or intensity) for the phototherapy device. This erythemally weighted intensity can be used in calculations related to dosage (i.e., Dose = Intensity x Time).

According to the ISO standard, 1 Standard Erythema Dose (SED) is equivalent to an erythemal effective radiant exposure (EERE) of 10 mJ/cm². Radiation sources that have the same absolute intensity can have a significant difference in exposure time needed to achieve 1 SED, even within the relatively narrow optimal wavelength range for maximum phototherapy efficacy (e.g., 298 nm-307 nm) because of the weighting factor applied to the absolute measured intensity of each wavelength.

### Skin Exposure

Phototherapy treatment of dermatologic disorders can consist of one or more individual treatment sessions using a device that delivers a dose of UV radiation. Because exposure to UV radiation is thought to be damaging to skin tissue and may be related to other conditions, safety of a phototherapy session can be increased by reducing or minimizing of total UV exposure. Devices can be sized and shaped to expose a small targeted area of skin (e.g., "spot-treat") or almost the entire skin surface (e.g., "full body"). Full body exposure to UV radiation can be reserved for severe cases of psoriasis that present lesions over the majority of the skin surface. Typically, phototherapy treatment is used as a as a spot treatment that exposes only the affected areas of the skin to UV radiation. For example, the skin lesions (scaly patches and plaques) that characterize psoriasis may be irradiated with a phototherapy device while avoiding the surrounding skin. This treatment protocol is typically selected to limit total UV exposure and relies primarily on a local immune suppression response rather than a systemic immune suppression response. However, such spot treatments limit three other mechanisms that provide a therapeutic benefit for the dermatological disorder: calcitriol production, vitamin D₃ production, and systemic immune response).

The amount of calcitriol, vitamin D₃, and systemic immune response produced within the UVB range is expected to be directly related to the total surface area of the skin exposed during a treatment. Increasing surface area of the skin exposed to UVB will therefore increase all of these responses, thereby increasing treatment efficacy while minimizing total UV exposure to any one area of the body because full body exposure does not require the intensity necessary for spot treatment. The effectiveness of this method can be magnified using a focused UVB range. For example, a phototherapy device that emits the majority of total UV output within the wavelength range 298 nm to 307 nm is consistent with the combination phototherapy action spectrum (Figure 16) and, therefore, will produce significantly more calcitriol, vitamin D3, and systemic immune response using significantly less total UV radiation than existing phototherapy technologies that are focused on delivering phototherapy to select areas of the body. For example, the present technology can distribute this focused energy evenly across a large surface area of the skin to improve efficacy of the treatment, while simultaneously reducing the total UV radiation to any one area.

A high-energy or low-energy device that emits focused UVB (298 nm-307 nm) can also be designed to "spot-treat" specific affected areas and would still provide maximum production of calcitriol, vitamin D3, local immune response, and systemic immune response compared to other non-focused UV devices. However, a dramatic improvement to efficacy using focused UV (298 nm-307 nm) is obtained through maximizing skin surface exposure during each phototherapy session.

It is thought that the minimum threshold of skin surface area that needs to be exposed to provide the systemic therapeutic benefit is about 30%. There is thought to be a direct correlation between percentage of skin surface area exposed (30%-100%) during a treatment session and overall treatment efficacy. Exposing at least 30% of a patient's total skin surface area to a focused UV range (298 nm-307 nm) during a single phototherapy session would allow efficacious treatment of dermatologic disorders, even without direct UV exposure to affected areas. This can be accomplished with a high-energy device, which easily treats large surface areas. Low-energy devices can also be configured to include larger arrays of radiation sources to provide for the treatment of large areas (e.g., a mat, jacket, or blanket). Alternatively, low-energy phototherapy devices that are smaller in scale can be used multiple times at various locations on the patient's body during a single phototherapy session (e.g., as in a small pad). For example, a patient with psoriasis lesions on the scalp can still be successfully be treated with a focused UV (298 nm-307 nm) device that exposes only skin from the shoulder down.

### Defining Dosage

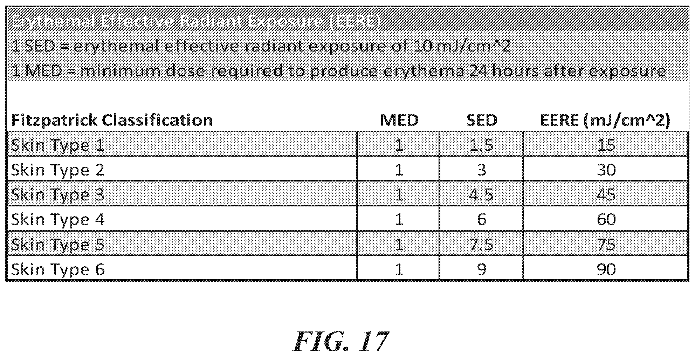

As discussed above, the Standard Erythema Dose (SED) is a standardized measurement of erythemogenic UV (not to be confused with the Minimal Erythema Dose (MED) used in phototherapy treatment). Determining the appropriate dose for treatment is based on the constitutive skin color of the patient, which can be expressed as a Fitzpatrick Skin Type 1-6. Skin type can also be determined by answering a series of questions related to the Fitzpatrick Skin Type scale (e.g., on an automated user interface or manually provided), determined automatically using a sensor or detector that measures the reflectance and/or absorption of a patient's skin, and/or determined using a grid that allows a patient or clinician to match the patient's skin tone to predetermined skin characteristics (e.g., fair, bums quickly; bums moderately; tans easily, etc.) and/or skin images of colors. In other embodiments, the patient's skin type can be determined automatically using other sensors and/or through automated and/or manual questionnaires or charts. The skin type is used to calculate 1 Minimal Erythema Dose (1 MED) is the amount of Erythemal Effective Radiant Exposure (EERE expressed in mJ/cm²) needed to produce a slight pink coloration of the skin within 24 hours. Because MED takes into consideration the skin type of the patient and the amount of EERE relative to that skin type, a "standard" phototherapy dose can be represented as a decimal of MED for all skin types. For example, a standard phototherapy dose for treatment with a device may be selected to be a constant 0.75 MED (or 75% of 1 MED) for all skin types. With 0.75 MED as the constant, the amount of EERE (mJ/cm²) becomes a variable that is adjusted according to skin type to achieve .75 MED. The exact amount of EERE needed to achieve 1 MED for each skin types (i.e., Skin Types 1-6) is expected to lie between 15 mJ/cm² to 90 mJ/cm², equivalent to 1.5 SED to 9 SED. The relationship between skin type, MED, SED, and EERE is reflected in Figure 17.

Skin type and MED can be determined using an instrument that measures skin reflectance or with information obtained from a questionnaire. Because skin reflectance instruments typically come in direct contact with the skin, such instruments can integrated into an LED array as part of a low-energy phototherapy system. In high-energy phototherapy systems, skin reflectance instruments can be incorporated into the system such that skin reflectance, skin type, and MED can be determined before treatment begins. With both high-energy and low-energy systems, a questionnaire could be administered and skin type determined before the treatment begins.

The UV dose for treatment of dermatologic disorders can be selected such that it produces significant efficacy without side effects. A phototherapy device that emits more than 75% of total UV output within the wavelength range 298 nm to 307 nm can be both effective for the treatment of dermatologic disorders and avoid side effects. However, a dosage range is needed to provide guidance for avoiding side effects and providing good efficacy. Because MED takes several variables into consideration, dosage provided by a phototherapy device can be expressed as a decimal MED constant. For example, a phototherapy device with focused UV range (e.g., 298 nm-307 nm) can have a dosage range of 0.2 MED (20% of 1 MED) to 0.9 MED (90% of 1 MED). Within this dosage range, 0.2 MED is expected to be the least efficient, but also have a relatively lower risk of side effects caused by skin exposure to UV, whereas 0.9 MED is expected to be the most efficient. As dosage is increased there is an equal increase in the level of UV exposure. Therefore, in certain embodiments the dosage can be selected to have an equal balance of UV exposure and efficacy, which would be about 0.55 MED. In other embodiments, the dosage can be higher or lower than 0.55 MED depending on the phototherapy device used, the type of efficacy and UV exposure desired, and a patient's skin type.

### Combining MED Dosage with Skin Exposure

Combining dosage with skin exposure percentage can be used to adjust the balance of UV exposure and efficacy. As described above, the efficacy of the phototherapy treatment is expected to be a function of, at least in part, the amount of surface area of the patient's skin exposed to UV radiation and the degree of MED applied, with more skin exposure and higher levels of MED expected to provide a more effective therapy. In certain embodiments, for example, the dosage range for a phototherapy treatment session using a focused UV range (298 nm-307 nm) includes a maximum dose of 0.9 MED to 100% of a patient's skin surface area to a minimum dose of 0.2 MED to 30% of a patient's skin surface area. In other embodiments, more or less of the patient's skin can be exposed and/or the MED range can be higher or lower.

It is expected that the percentage of skin exposure contributes to efficacy of the phototherapy, but does not necessarily impact the risk of side effects. For example, if dosage is held constant (e.g., at 0.55 MED) and skin exposure percentage is increased, the efficacy is expected to increase without increasing the risk of side effects. Accordingly, as long as the phototherapy dose is 0.2 MED to 0.9 MED and skin exposure percentage is greater than 30%, it is possible to trade dose and exposure percentage to achieve a desired balance of efficacy, while mitigating the risks of potential side effects. That is, phototherapy dosages and the resultant efficacy can be selected based on the total skin exposure (e.g., 30%-100%) and the percentage of 1 MED (e.g., 20%-90%), and these two parameters (i.e., percentage skin exposure and MED dose) can be selected based on the desired result and patient-specific needs (e.g., specific indication, skin type, etc.).

It is also possible to maintain a constant efficacy by varying skin exposure percentage relative to MED dosage. Accordingly, increasing the skin surface area exposed can lower the necessary MED dosage to achieve the same level efficacy. For example, the same efficacy in a phototherapy session can be achieved with a 0.2 MED dose and 100% skin exposure as with a 0.4 MED dose and 50% skin exposure. Similarly, phototherapy treatment sessions can have the same efficacy with (a) a 0.4 MED dose and 60% skin exposure as with a 0.8 MED dose and 30% skin exposure, or (b) a 0.9 MED dose and 40% skin exposure as with a 0.45 MED dose and 80% skin exposure. It is thought that the MED dosage is the parameter that best controls the side effects of the phototherapy session (e.g., exposure to UV radiation), whereas the percentage of skin exposure does not. Therefore, in various embodiments, the selected dosage includes an increased percentage of skin exposure and a decreased MED dosage.

### Dosage Tables

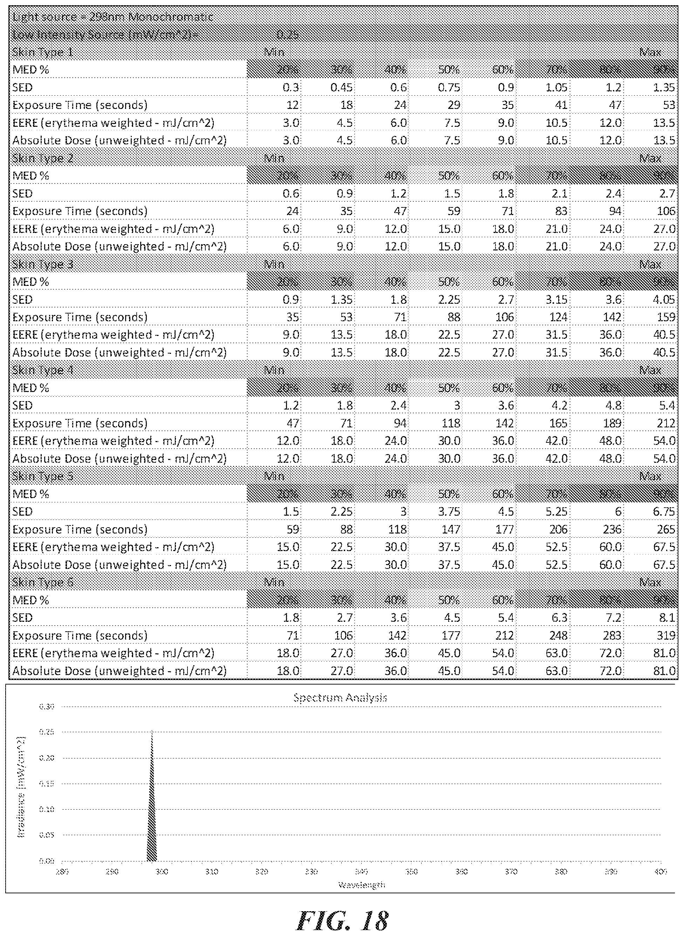

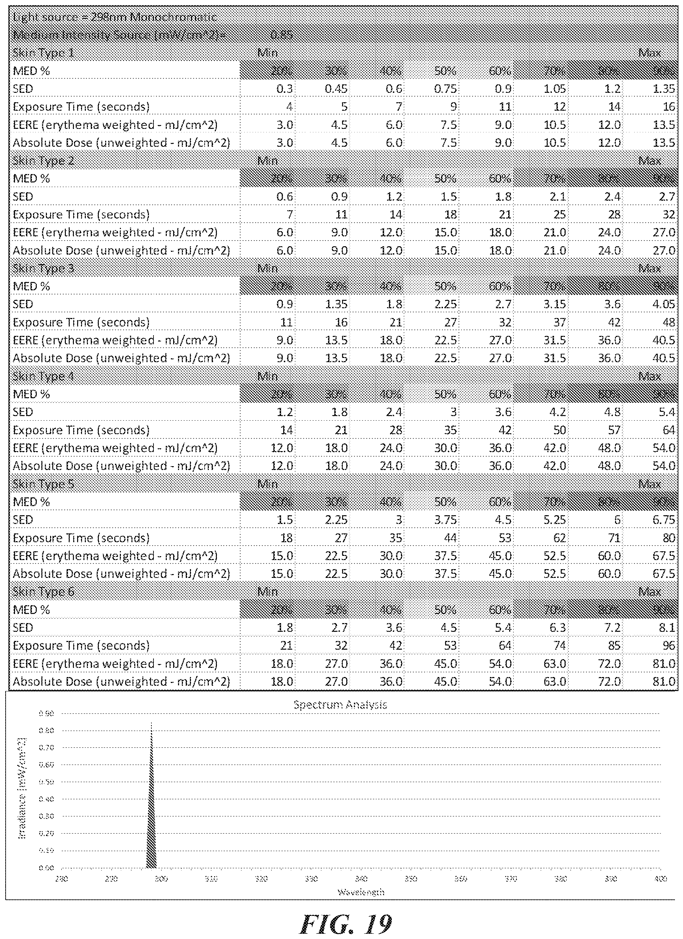

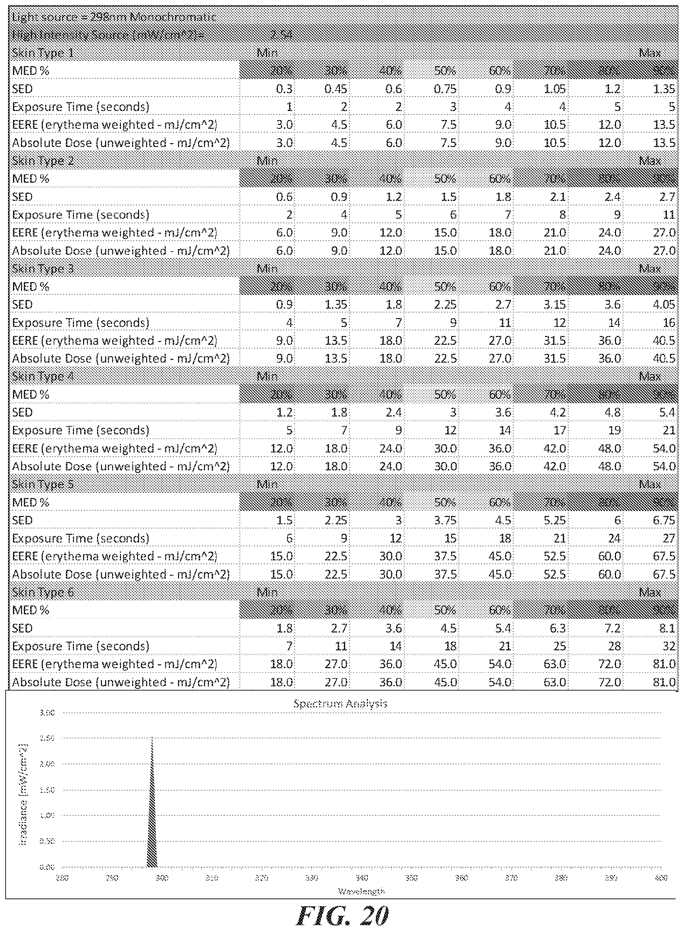

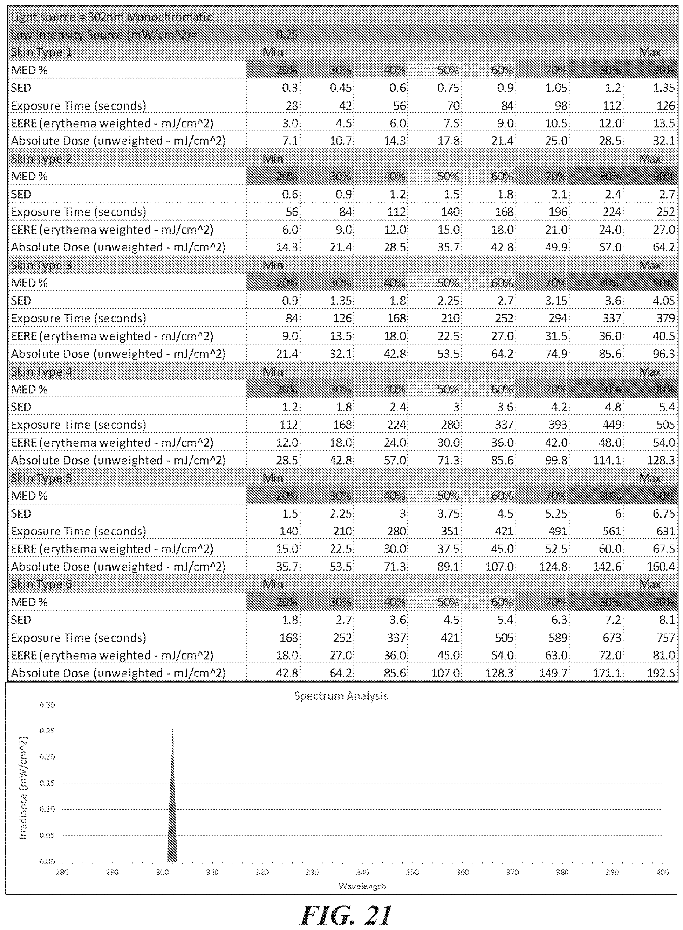

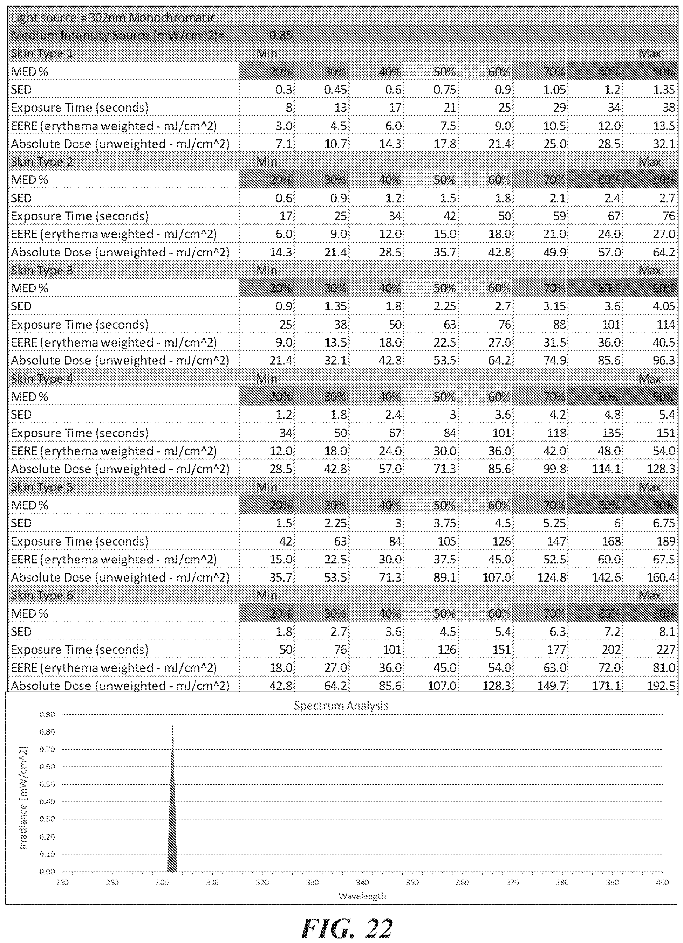

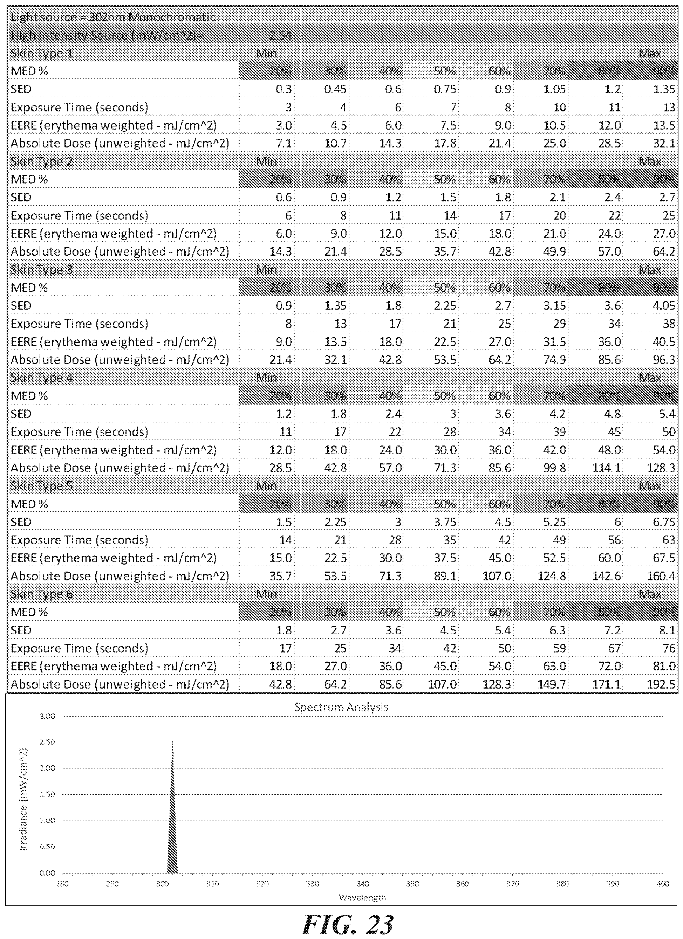

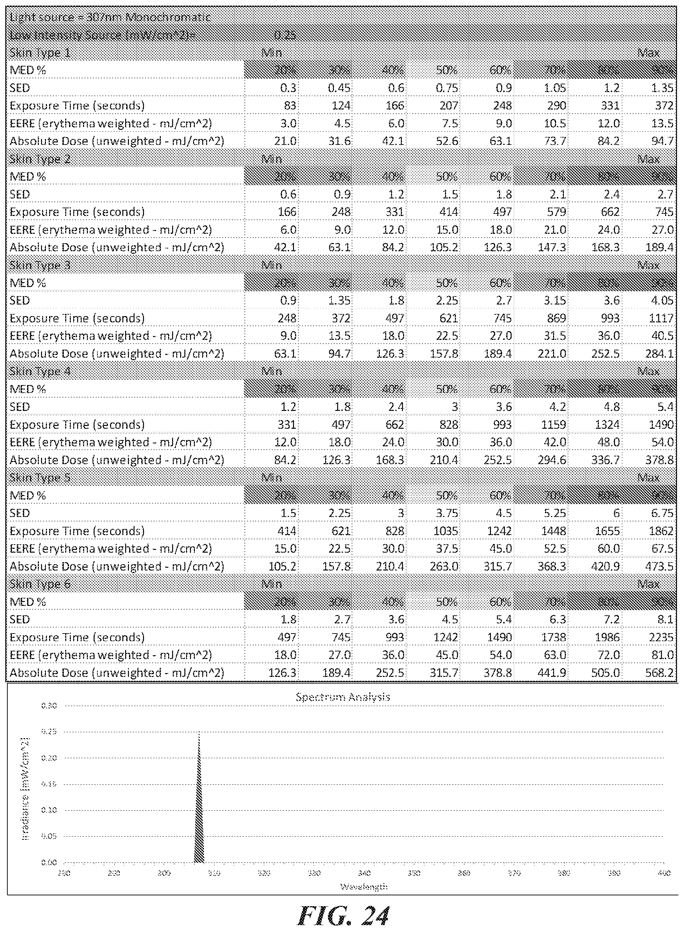

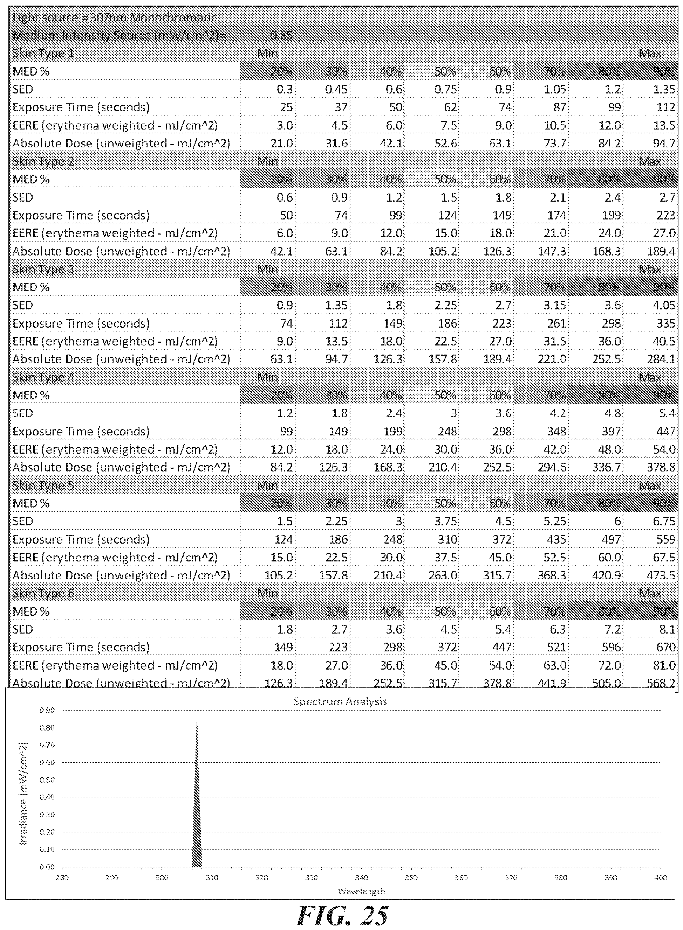

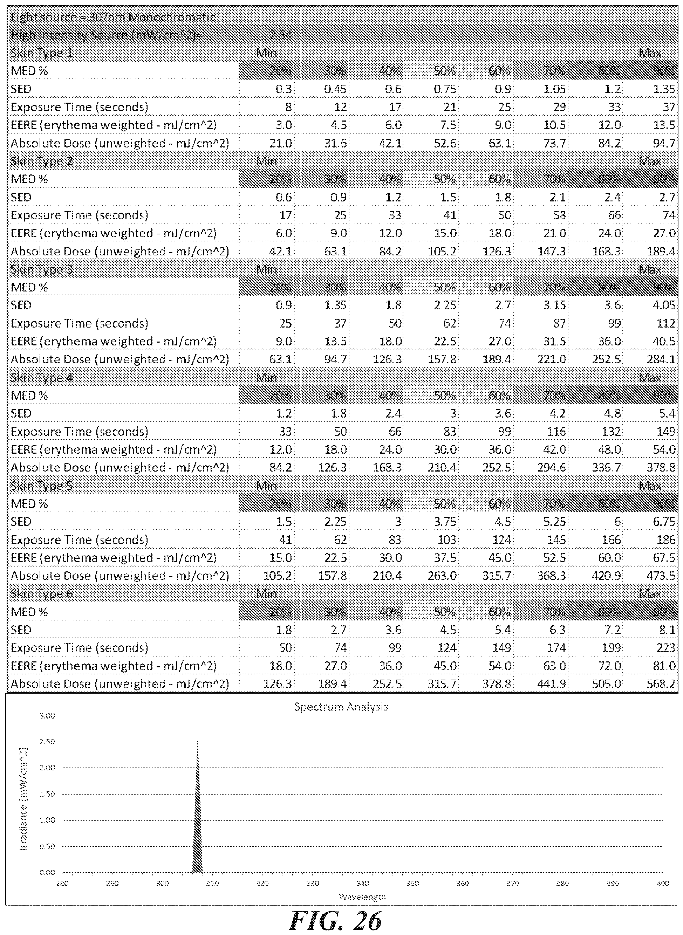

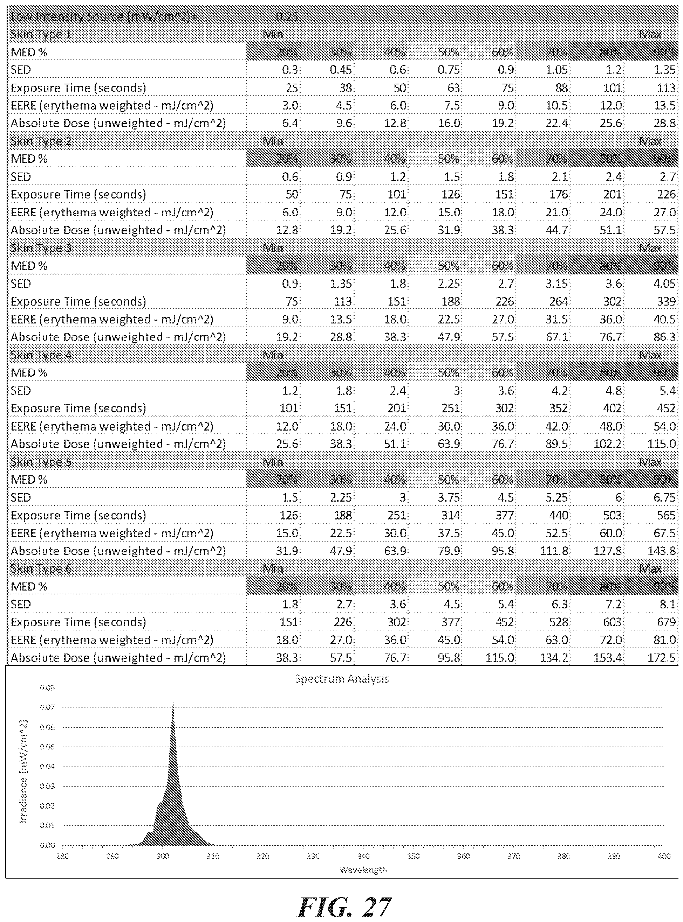

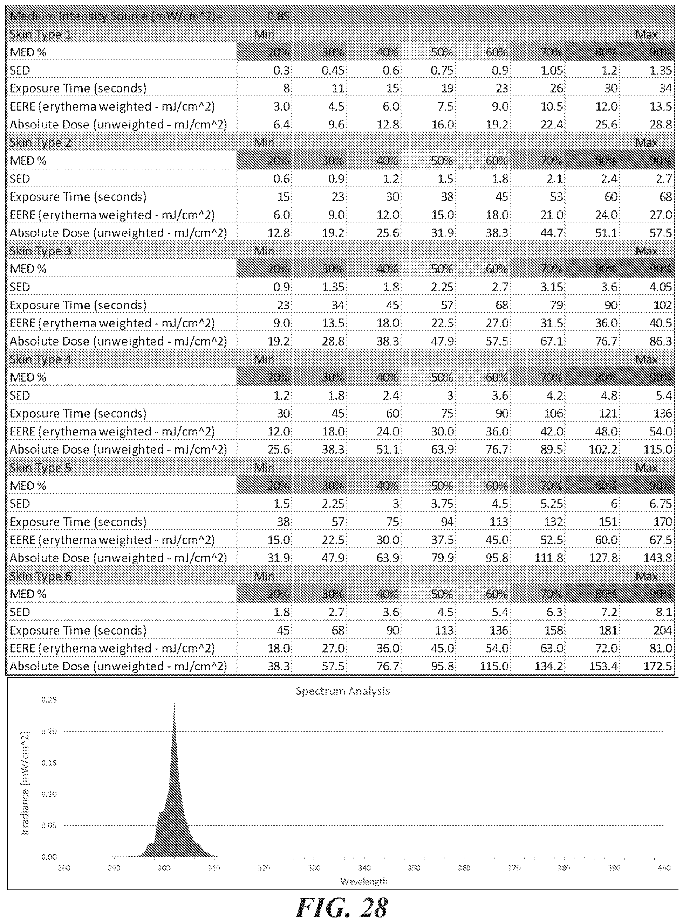

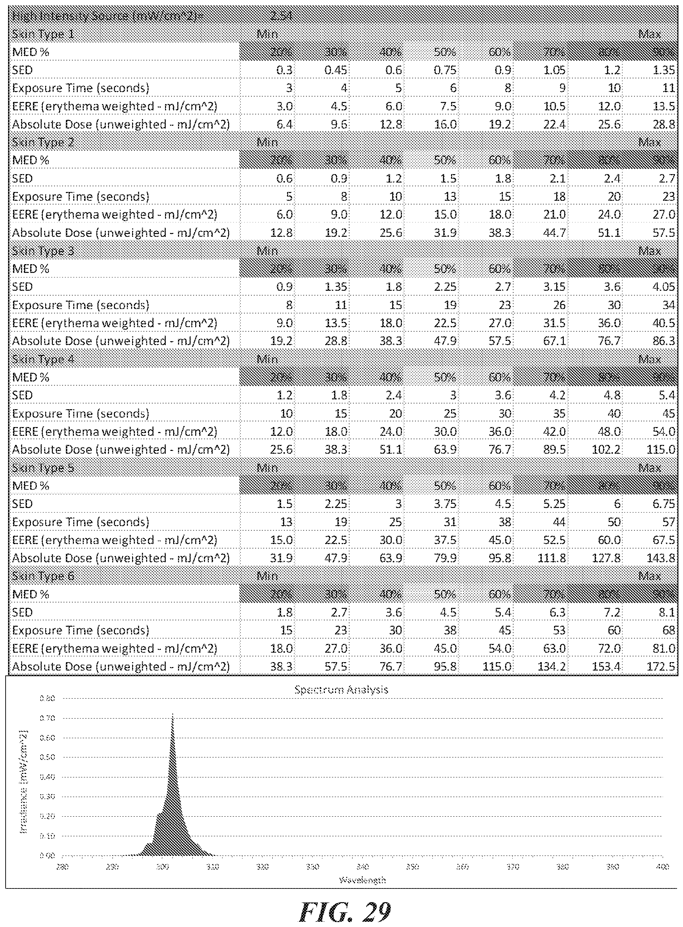

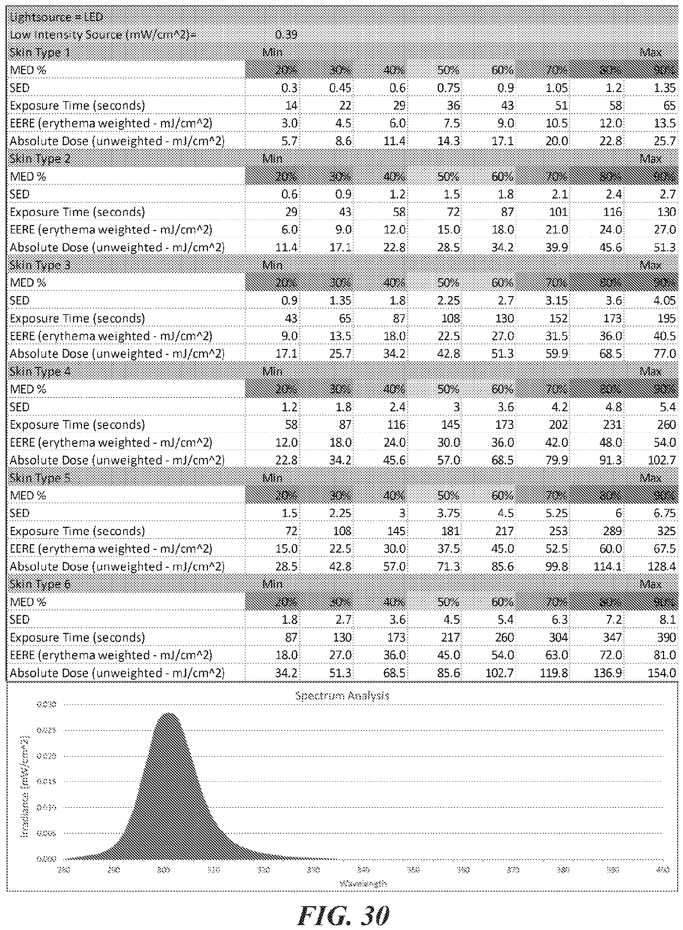

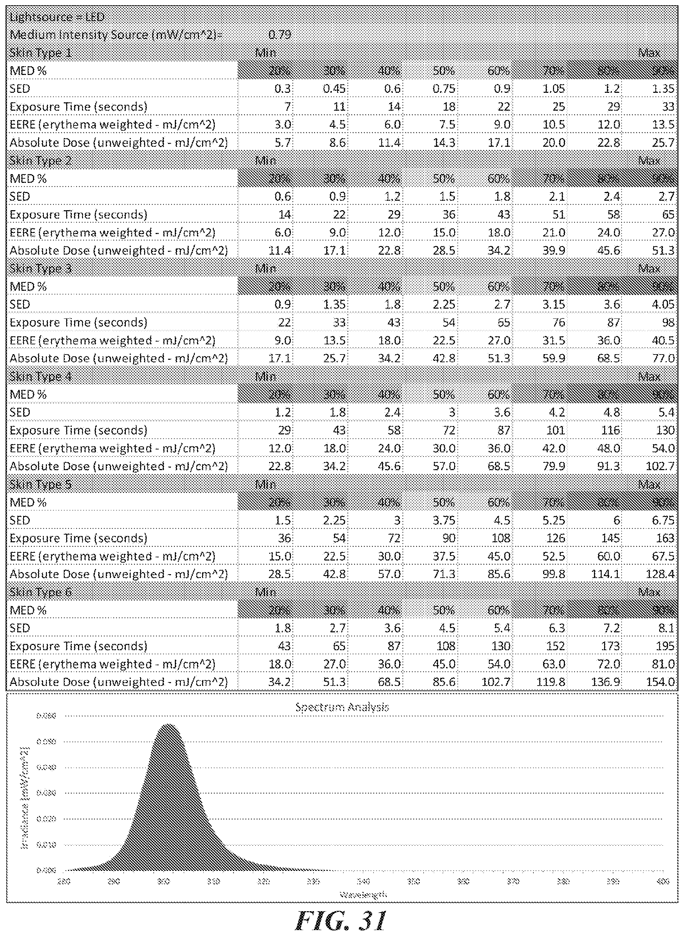

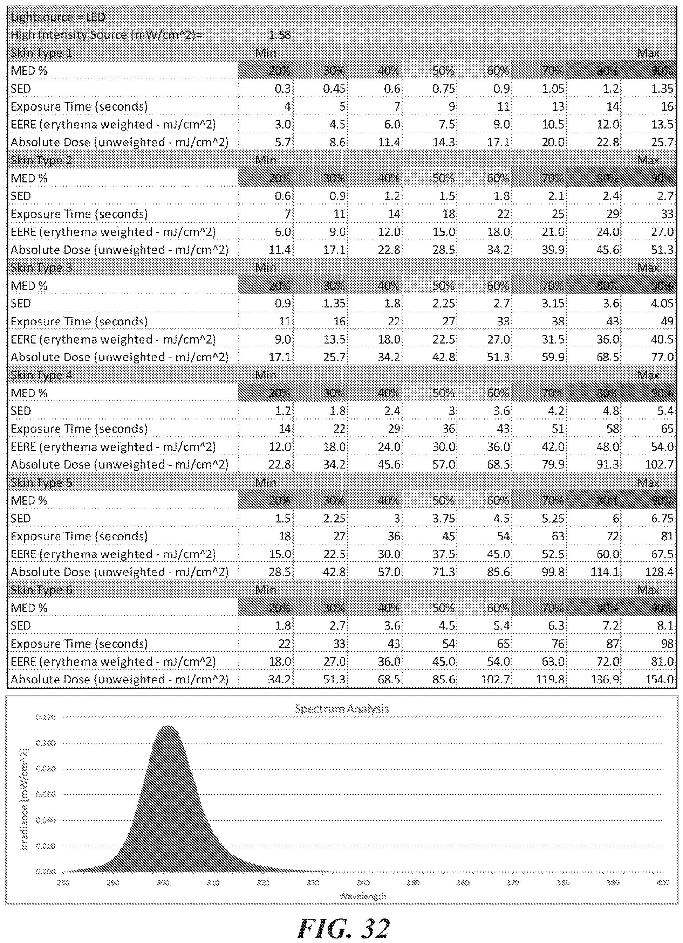

In practice, the parameters of phototherapy sessions for treating dermatologic disorders can be determined using dosage tables or charts for a selected phototherapy device with known or measured spectrum irradiance values and a selected MED dosage (e.g., 0.2 MED to 0.9 MED). For example, these dosage charts can be used to determine the SED, exposure time (e.g., seconds), absolute dose (mJ/cm²), and EERE (mJ/cm²) for each Fitzpatrick skin type for the selected phototherapy device. In certain embodiments, for example, a phototherapy device with focused UV range (e.g., 298 nm-307 nm) can have an MED dosage range of 0.2 MED (20% of 1 MED) to 0.9 MED (90% of 1 MED). Given this wavelength and MED dosage range, the calculation of exposure time, absolute dose and EERE can be calculated based on the device intensity and exact spectrum irradiance of the light source. This information can then be used to create a dosage chart showing the dosage range for each skin type for a specific phototherapy treatment device. Figures 18-32 illustrate such dosage tables for five phototherapy devices with (a) different spectrum irradiances: a 298 nm monochromatic UV source (Figures 18-20), a 302 nm monochromatic UV source (Figures 21-23), a 307 nm monochromatic UV source (Figures 24-26), a 302 nm filtered metal halide UV source (Figures 27-29), and a 301 nm LED (Figures 30-32); and (b) three different device intensity examples (i.e., low, medium, high) for each UV source. Using these dosage charts, a clinician can understand the range of operating parameters for a focused UV phototherapy device and select the desired parameters for a phototherapy session for a specific patient, varying the MED dosage accordingly. As shown in Figure 20, for example, the 298 nm monochromatic high intensity UV source can deliver 0.2 MED to a patient having Skin Type 1 in a phototherapy session having a totally exposure time of just 1 second and an absolute irradiance of only 3.0 mJ/cm². As shown in Figure 24, using the 307 nm monochromatic low intensity UV source to deliver 0.9 MED to a patient having Skin Type 6 requires a phototherapy session of 37.25 minutes and has an absolute irradiance of 568.2 mJ/cm².

### VII. Selected Embodiments of Phototherapeutic Systems

Figure 33 is an isometric view of a high-energy phototherapeutic apparatus or system ("system 3300") for focused UV radiation configured in accordance with an embodiment of the present technology. The system 3300 includes a plurality of focused UV radiation fixtures or assemblies 3310 ("radiation assemblies 3310") that emit energy within a predetermined wavelength range (e.g., about 298-307 nm, 298-304 nm, 300-305 nm, etc.). For example, the system 3300 can be used to emit UVB radiation within the optimum wavelength range shown in the combination phototherapy action spectrum of Figure 16. Each radiation assembly 3310 can emit energy having a substantially similar wavelength and similar intensity as the other radiation assemblies 3310 of the system 3300, or the emitted wavelengths and intensities of the individual radiation assemblies 3310 within the system 3300 may differ. In the illustrated embodiment, the radiation assemblies 3310 are carried by two housings, arms, or columns (identified individually as a first column 3330a and a second column 3330b, and referred to collectively as columns 3330) that are mounted on or otherwise attached to a pedestal or base 3332, and the radiation assemblies 3310 are directed generally inward toward a central portion 3334 of the base 3332. The base 3332 and the columns 3330 together define an irradiation zone in which a human can be exposed to focused UVB energy emitted by the radiation assemblies 3310. When a user (e.g., a human) stands on or is otherwise positioned at the central portion 3334 of the base 3332, the radiation assemblies 3310 can irradiate the user's skin to treat dermatological disorders, stimulate vitamin D production in the skin, and/or treat other indications that may benefit from exposure to the predetermined wavelength range. In various embodiments, the central portion 3334 of the base 3332 and/or the columns 3330 may rotate relative to each other to expose all sides of the user's body to the energy emitted by the radiation assemblies 3310.

The system 3300 can provide an at least substantially uniform distribution of irradiation intensity by taking into account various features of the system 3300. For example, in the embodiment illustrated in Figure 33 the radiation assemblies 3310 in the first column 3330a can be vertically offset from the radiation assemblies 3310 in the second column 3330b to prevent the irradiation from radiation assemblies 3310 of the first column 2030a from directly overlapping the irradiation from the radiation assemblies 3310 of the second column 3330b. For example, the radiation assemblies 3310 in the first column 3330a can be offset from radiation assemblies 3310 in the second column 3330b by about one radius of an individual radiation assembly 3310. This staggering of the radiation assemblies 3310 can provide a more uniform intensity of irradiation along the length of the columns 3330 and prevent certain areas of a user's skin from being exposed to more irradiation than others. In other embodiments, the system 3300 can include different features and/or other radiation assembly configurations to enhance the uniformity of the radiation emitted by the radiation assemblies 3310 and/or manipulate the direction in which the radiation is projected. For example, one or more lenses can be positioned forward of one or more of the radiation assemblies 3310 and configured to bend the light in a manner such that the light is evenly distributed across the irradiation zone or a portion thereof. In further embodiments, uniform emissions can be accomplished by selecting the distance the patient must be positioned away from the radiation assemblies 3310 to receive substantially uniform irradiation distribution, and/or the output of the system 3300 may be adjusted by changing the energy input, the number of lamps, lens specifications, and/or filtration parameters.

In further embodiments, the system 3300 can include columns 3330 with fewer than or more than the eight radiation assemblies 3310 shown in Figure 33 (e.g., one radiation assembly, two radiation assemblies, four radiation assemblies, nine radiation assemblies, etc.), a single column 3330 of radiation assemblies 3310, more than two columns 3330 of radiation assemblies 3310 (e.g., four columns, six columns, etc.), and/or the radiation assemblies 3310 can be arranged in other suitable configurations. For example, the radiation assemblies 3310 can be carried by a housing that at least substantially encloses the irradiation zone and directs radiation inward toward an enclosed space defined by the housing.

The system 3300 can emit high intensity focused UVB radiation to provide therapeutic effects on dermatological disorders or other indications, and/or facilitate vitamin D production in the skin during relatively short phototherapy sessions. For example, the apparatus 3300 can provide a sufficient amount of irradiation during a phototherapy session (e.g., 30 seconds, 1 minute, 2 minutes, 5 minutes, etc.) to stimulate the production of a weekly or monthly dose of vitamin D. In various embodiments, the exposure time of each phototherapy session can be selected based on the on the user's skin type and/or the intensity of the radiation assemblies 3310. The user's skin type can be determined based on the Fitzpatrick skin type or a detector that measures skin reflectance and/or absorption. More specifically, the user's skin type can also be determined by answering a series of questions related to the Fitzpatrick Skin Type scale (e.g., on an automated user interface of the system 3300), determined automatically using a sensor or detector on the housing of the system 3300 and/or operably coupled to the system 3300 that measures the reflectance, absorption, and/or other features related to skin type, and/or determined using a grid that allows the user or clinician to match the patient's skin tone to predetermined skin characteristics (e.g., fair, burns quickly; bums moderately; tans easily, etc.) and/or skin images of colors. In other embodiments, the patient's skin type can be determined automatically or manually using other suitable mechanisms and methods for determining skin type. For example, the lighter the user's skin tone, the less exposure time necessary to obtain the desired level of UVB exposure in the user's skin or the less exposure time allowed to avoid overexposing the user's skin. As another example, the higher the intensity of the energy provided by the system 3300, the less exposure time necessary to obtain the desired irradiation for phototherapy. In certain embodiments, the amount of UVB emissions provided to each user can be selected using the dosage tables shown in Figures 18-32.

As shown in Figure 33, each radiation assembly 3310 can include a UV radiation source 3312, a reflector 3336 partially surrounding the UV radiation source 3312, and a filter 3338 forward of the radiation source 3312. The radiation source 3312 can emit high energy (e.g., UV light), and at least some of the energy can contact the reflector 3336 (e.g., a mirrored substrate or coating) before exiting the radiation assembly 3310. The reflector 3336 can divert or otherwise direct the light forward toward the filter 3338 where light within a predetermined bandwidth (e.g., 6 nm, 8 nm, 16 nm, etc.) can exit the radiation assembly 3310. In certain embodiments, the reflector 3336 is curved around the radiation source 3312 such that the light emitted by the radiation source 3312 at least substantially collimates upon contact with the reflector 3336. The collimated beam of light can then travel forward toward the filter 3338, and pass through the filter 3338 at the same angle of incidence (e.g., about 0°) to provide substantially uniform filtering of the light. In other embodiments, the radiation assemblies 3310 may not include the reflector 3336, and/or the radiation assemblies 3310 can include other features that collimate the radiation emitted from the radiation sources 3312. In certain embodiments, the filter 3338 can be a lens that uniformly distributes the energy before it is emitted toward the user in the central portion 3334. In other embodiments, the uniform light distribution lens may be separate from the filter 3338.

The radiation source 3312 can include a metal halide lamp, which is a type of high-intensity discharge ("HID") lamp that generates light by producing an electric arc through a gaseous mixture between two electrodes in an arc tube or envelope. The arc length (i.e., about the distance between the electrodes) of the metal halide lamp can be relatively small with respect to radiation assembly 3310 as a whole such that the metal halide lamp acts similar to a point source to facilitate collimation of the light. In other embodiments, the metal halide lamp can have larger or smaller arc lengths depending on the configuration of the metal halide lamp and the sizing of the other components of the radiation assembly 3310 (e.g., the reflector 3336). In other embodiments, the radiation source 3312 may include different types of high-energy UVB-emitting sources, such as mercury arc lamps, pulse and flash xenon lamps, and fluorescent lamps.

When using metal halide lamps as the radiation source 3312, the gas mixture in the arc tube of the metal halide lamp can be selected to increase the UVB content of the emissions of the metal halide lamp. For example, the gas mixture can be doped to generate about 6% of the total emissions in the UVB range (e.g., about 280-315 nm) in comparison to normal tanning bed lamps that have about 1% of their emissions in the UVB range. The increased UVB content of the emissions can increase the intensity of the UVB emitted by the radiation assembly 3310, and therefore may decrease the overall exposure time necessary to achieve a desired phototherapy. Based on test data, it is believed that large portions of the emissions of doped metal halide lamps have wavelengths of about 300-305 nm. As discussed above with respect to Figure 16, the combination phototherapy action spectrum suggests that an optimal wavelength range for treatment of dermatological disorders is about 298-307 nm. Accordingly, metal halide lamps are uniquely suited for promoting vitamin D production in the skin and immune responses for dermatological disorders, and may require less filtering than other types of UV radiation sources.

The filter 3338 can be a narrow pass filter that prevents UVB radiation outside of a predetermined bandwidth from exiting the radiation assembly 3310. In certain embodiments, the filter 3338 can include a substrate (e.g., glass, plastic, etc.) and at least one interference coating applied to the substrate. The coating can be sprayed onto the substrate and/or otherwise disposed on the substrate using methods known to those skilled in the art. Substrates and interference coatings that provide at least some filtering of UV radiation outside of a predetermined spectrum are available from Schott of Elmsford, New York. In various embodiments, other portions of the radiation assemblies 3310 can include interference coatings and/or other filtering features that block at least some radiation outside of the desired wavelength spectrum. For example, an absorption filter can be incorporated into the envelope of a metal halide lamp (e.g., metal additives can be incorporated into the quartz of the lamp). The combination phototherapy action spectrum described above with reference to Figure 16 can be used to determine the most efficient wavelength for phototherapy, and a narrow pass filter can be designed or selected to emit radiation centered at the predetermined wavelength. For example, in certain embodiments, the filter 3338 can at least substantially block UVB radiation outside of a predetermined spectrum (e.g., about 298-307 nm). In other embodiments, the filter 3338 can at least substantially block UVB radiation outside of a selected bandwidth (e.g., a 4 nm spectrum, a 6 nm spectrum, an 8 nm spectrum, a 12 nm spectrum, a 16 nm spectrum, etc.), and/or the spectrum can be centered around other suitable wavelengths for treating dermatological disorders and/or producing vitamin D (e.g., 298 nm, 300 nm, 302 nm, etc.).

The concentrated UVB radiation provided by the system 3300 can deliver a large amount of UVB radiation within the desired wavelength range (e.g., shown in Figure 16) within a relatively short phototherapy session (e.g., less than 10 minutes, less than 5 minutes, less than 2 minutes, less than 1 minute, etc.). The UVB radiation can be distributed in a substantially uniform emission pattern such that the exposed area of the user's skin (i.e., the treatment area) is exposed to a substantially uniform intensity of light. The dosage provided to each user can be selected based on the dosage tables described above with respect to Figures 18-32.

Figure 34 is an isometric view of a low-energy phototherapeutic apparatus or system ("system 3400") for focused UV radiation configured in accordance with another embodiment of the present technology. The system 3400 can include a wearable substrate 3410 and a plurality of low-intensity radiation sources 3420 (e.g., 3 Watts or less), such as a plurality of LEDs. As used herein, a wearable substrate refers to an article or apparatus that can come in close proximity to a patient's skin (e.g., within 3 cm of the patient's skin) such that the patient comes in close proximity to the radiation sources 3420. In the embodiment illustrated in Figure 34, for example, the wearable substrate 3410 is a blanket or pad that a patient can lay on top of or under. In other embodiments, the wearable substrate 3410 may be other items, such as bands that wrap around portions of a patient's body (e.g., a patient's leg, arm, torso, wrist, etc.), sleeves, clothing (e.g., tightly fitting shirts or pants), and/or other articles that can carry the low-intensity radiation sources and can be held in close proximity to the patient's skin. The wearable phototherapy system 3400 can provide a substantially uniform and constant level of radiation intensity across the portion of the wearable substrate 3410 including the radiation sources 3420. This allows phototherapy to be delivered to selective and scalable treatment areas.

The radiation sources 3420 of the system 3400 can be arranged on the wearable substrate 3410 in tightly packed arrays. In various embodiments, the radiation sources 3420 are spread evenly across the wearable substrate 3410 (e.g., as shown in Figure 34), whereas in other embodiments the radiation sources 3420 are spaced in specific sections or unevenly distributed across the wearable substrate 3410. The radiation sources 3420 can be LEDs that emit light with relatively monochromatic wavelength emissions (e.g., 298 nm, 300 nm, 302 nm, 305 nm, etc.) or at a plurality of different wavelengths within a predetermined narrow bandwidth (e.g., 10 nm bandwidth, 7 nm bandwidth, 5 nm bandwidth, etc.) suitable for treating dermatological disorders, vitamin D deficiency, autoimmune disorders, and/or other indications. For example, the wavelengths of the LEDs can be selected using the methods and action spectra described above with respect to Figures 1-16. In certain embodiments, the LEDs can emit wavelengths between 298 nm and 307 nm. In other embodiments, the LEDs can have one or more different wavelengths, such as wavelengths ranging from 295 nm to 310 nm or therebetween. The monochromatic output of the LEDs may reduce or eliminate the amount of filtering necessary to provide UVB radiation within a predetermined spectrum. Suitable LEDs are available from, for example, Sensor Electronic Technology, Inc. of Columbus, South Carolina.

The individual radiation sources 3420 can also include one or more lenses 3430 (identified individually as a first lens 3430a and a second lens 3430b). Individual lenses, such as the first lens 3430a, can be positioned over each individual radiation source 3420. In other embodiments, a larger lens, such as the second lens 3430b, can extend over two or more of the radiation sources 3420 (e.g., all of the radiation sources 3420 on the wearable substrate 3410. In certain embodiments, the larger second lens 3430b can be used in conjunction with the individual first lenses 3430a. In further embodiments, the system 3400 can include other features that spread the emitted light at least substantially evenly across a portion of the wearable substrate 3410 or the entire surface area of the wearable substrate 3410.

The intensity of the array of radiation sources 3420 can be selected by adjusting various parameters of the radiation sources 3420 and the array of the radiation sources 3420. For example, the intensity of the radiation source array can be increased by increasing the input energy delivered to the radiation sources 3420 (e.g., by changing the power source or controls thereon), increasing the quantity of the radiation sources 3420 per unit area, decreasing the distance between the radiation sources and the treatment area on the patient (e.g., 0-3 cm, within 4 cm, within 5 cm, etc.), decreasing the degree of light spreading of the lens(es) 3430 on the radiation sources 3420, and/or changing other features of the radiation source array that impact the radiation intensity. Conversely, the intensity of the radiation source array can be decreased by decreasing the level of energy delivered to the radiation sources 3420, decreasing the quantity of the radiation sources 3420 per unit area, increasing the distance between the radiation sources 3420 and the treatment area on the patient, increasing the degree of light spreading of the lens(es) 3430, and/or changing other features of the radiation source array that impact the radiation intensity.

As shown in Figure 34, the system 3400 can further include a controller 3450 operably coupled to the radiation sources 3420 on the wearable substrate 3410. The controller 3450 can be coupled to radiation sources 3420 via a wired connection line 3460 (e.g., an electrical cord) or via a wireless connection (e.g., Bluetooth, internet, intranet, etc.). The controller 3450 can be manipulated by an operator (e.g., a clinician, a technician, and/or the user) to activate and deactivate the system 3400, as well as adjust various parameters of the system 3400. These parameters can include, for example, the level of energy delivered to the radiation sources 3420. As described in further detail below, the controller 3450 can include various automated programs and algorithms that adjust the parameters of the system 3400. For example, the controller 3450 can adjust the dosage provided by the system 3400 using the dosage tables described above with respect to Figures 18-32.

In operation, the system 3400 can provide an at least substantially uniform distribution of irradiation intensity by taking into account various features of the system, such as the distance between the radiation sources 3420 and the treatment site on the patient, the spacing of the radiation sources 3420 with respect to each other, and/or the shape of the lenses 3430 on the radiation sources 3420. For example, the radiation source array can be arranged such that at least a major portion of emission patters of the individual radiation sources 3420 do not overlap each other. The lenses 3430 on the radiation sources 3420 can be used to expand or contract the emissions of the individual radiation sources 3420 such that they do not overlap each other. In certain embodiments, radiation sources 3420 are spaced apart by a distance that avoids any overlapping emissions, and therefore leaves some portions of the treatment area (e.g., the area of skin facing the wearable substrate 3410) unexposed from the emissions.

In various embodiments, the system 3400 can be configured such that the radiation sources 3420 remain at a constant distance from the treatment area during the phototherapy session to maintain the uniform exposure to the radiation sources 3420. Accordingly, the wearable substrate 3410 can be placed in direct contact with the treatment area. In certain embodiments, the system 3400 can include a sensor 3440 that indicates when the radiation sources 3420 are appropriately placed on the skin to confirm direct skin contact before and/or during operation of the system 3400 during a phototherapy session. The embodiment illustrated in Figure 34 includes a single sensor 3400. However, in other embodiments, the system 3400 can include a plurality of sensors 3440 spaced across the wearable substrate to confirm contact with the patient's skin.

In further embodiments, the sensor 3440 can include a detector that measures skin reflectance and/or absorption to automatically determine a patient's skin type before the phototherapy is applied. In other embodiments, the sensor 3440 can measure other characteristics related to skin type. As described above, this information can be used in determining the correct dosage to provide to the patient. The controller 3450 can then be used to adjust the parameters of the system 3400, such as phototherapy duration and energy input, in response to the measured skin type. In other embodiments, this information can be manually entered into the controller 3450. In further embodiments, skin type can be determined by answering questions a series of questions related to the Fitzpatrick Skin Type scale (e.g., on an automated user interface of the system 3400), using a grid that allows the user or clinician to match the patient's skin tone to predetermined skin characteristics (e.g., fair, bums quickly; bums moderately; tans easily, etc.) and/or skin images of colors, and/or using other suitable mechanisms and methods for determining skin type.

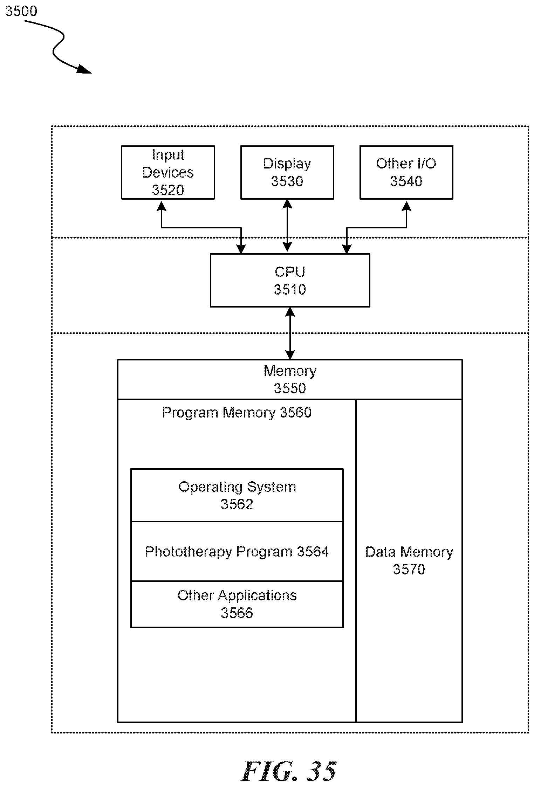

Figure 35 is a block diagram illustrating an overview of devices on which some implementations of the disclosed technology can operate. The devices can comprise hardware components of a device 3500 for selecting parameters for phototherapy sessions that may affect phototherapy dosage. This device 3500 may be a controller, such as the controller 3450 of Figure 34, that operates a phototherapy system (e.g., the phototherapy systems 3300 and 3400 described above with reference to Figures 33 and 34). Device 3500 can include, for example, one or more input devices 3520 providing input to a central processing unit ("CPU"; processor) 3510, notifying the CPU 3510 of actions. The actions are typically mediated by a hardware controller that interprets the signals received from the input device and communicates the information to the CPU 3510 using a communication protocol. The input devices 3520 include, for example, a receiver for receiving signals from sensors (e.g., skin contact sensors, distance or proximity sensors, skin reflectance or absorption detectors, other skin type sensors, etc.), a mouse, a keyboard, a touchscreen, an infrared sensor, a touchpad, a wearable input device, a camera- or image-based input device, a microphone, and/or other user input devices.

The CPU 3510 can be a single processing unit or multiple processing units in a device or distributed across multiple devices. The CPU 3510 can be coupled to other hardware devices, for example, with the use of a bus, such as a PCI bus or SCSI bus. The CPU 3510 can communicate with a hardware controller for devices, such as for a display 3530. The display 3530 can be used to display text and graphics. In some examples, the display 3530 provides graphical and textual visual feedback to a user, such as the parameters of a phototherapy session, a summary of indices detected by a detector coupled to the device 3500, and/or other suitable information. In some implementations, the display 3530 includes the input device as part of the display, such as when the input device is a touchscreen or is equipped with an eye direction monitoring system. In some implementations, the display 3530 is separate from the input device 3520. Examples of display devices are: an LCD display screen, an LED display screen, a projected, holographic, or augmented reality display (such as a heads-up display device or a head-mounted device), and so on. Other I/O devices 3540 can also be coupled to the processor, such as a network card, video card, audio card, USB, firewire or other external device, camera, printer, speakers, CD-ROM drive, DVD drive, disk drive, or Blu-Ray device.

In some implementations, the device 3500 also includes a communication device capable of communicating wirelessly or wire-based with a network node. The communication device can communicate with another device or a server through a network using, for example, TCP/IP protocols. Device 3500 can utilize the communication device to distribute operations across multiple network devices.

The CPU 3510 can have access to a memory 3550. A memory includes one or more of various hardware devices for volatile and non-volatile storage, and can include both read-only and writable memory. For example, a memory 3550 can include random access memory (RAM), CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory. The memory 3550 can include program memory 3560 for storing programs and software, such as an operating system 3562, a phototherapy program 3564, and other application programs 3566. The sympathetic tone analysis program 3564, for example, can include one or more algorithms for determining the parameters of a phototherapy system (e.g., the systems 3300 and 3400 described in Figures 33 and 34) to provide proper dosage for a patient, analyzing parameters of a system during a phototherapy session, and/or providing a recommendation for a specific therapy or specific parameters of a therapy that a clinician or other user can then adjust. The memory 3550 can also include data memory 970 including recorded data from a cardiac detector, patient data, algorithms related to sympathetic tone analysis, configuration data, settings, user options or preferences, etc., which can be provided to the program memory 3560 or any element of the device 3500.

Some implementations can be operational with numerous other general purpose or special purpose computing system environments or configurations. Examples of well-known computing systems, environments, and/or configurations that may be suitable for use with the technology include, but are not limited to, personal computers, server computers, handheld or laptop devices, cellular telephones, wearable electronics, tablet devices, multiprocessor systems, microprocessor-based systems, set-top boxes, programmable consumer electronics, network PCs, minicomputers, mainframe computers, distributed computing environments that include any of the above systems or devices, or the like.

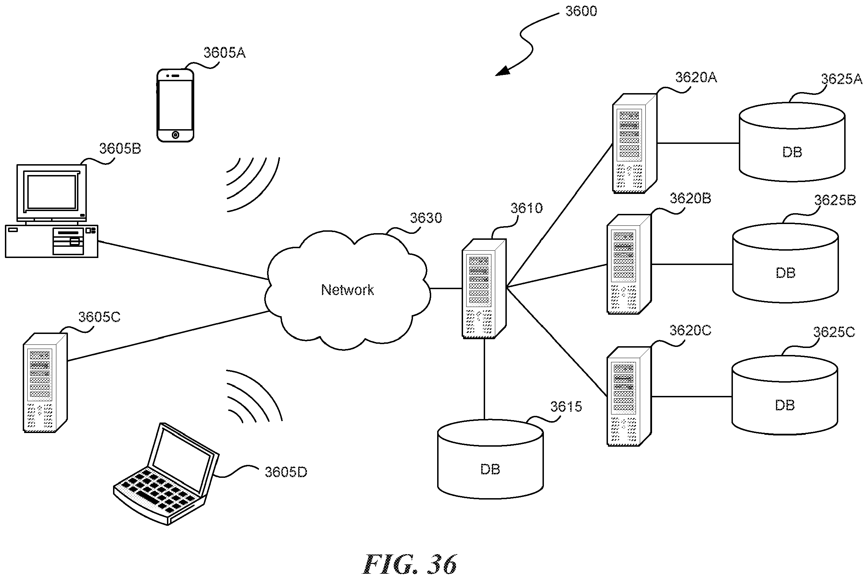

Figure 36 is a block diagram illustrating an overview of an environment 3600 in which some implementations of the disclosed technology can operate. The environment 3600 can include one or more client computing devices 3605A-D (identified collectively as "client computing devices 3605"), examples of which can include the device 3500 of Figure 35. The client computing devices 3605 can operate in a networked environment using logical connections through a network 3630 to one or more remote computers, such as a server computing device 3610.

In some implementations, server 3610 can be an edge server that receives client requests and coordinates fulfillment of those requests through other servers, such as servers 3620A-C. The server computing devices 3610 and 3620 can comprise computing systems, such as device 3500 (Figure 35). Though each server computing device 3610 and 3620 is displayed logically as a single server, the server computing devices 3610 and 3620 can each be a distributed computing environment encompassing multiple computing devices located at the same or at geographically disparate physical locations. In some implementations, each server 3620 corresponds to a group of servers.

The client computing devices 3605 and the server computing devices 3610 and 3620 can each act as a server or client to other server/client devices. The server 3610 can connect to a database 3615. The servers 3620A-C can each connect to corresponding databases 3625A-C. As discussed above, each server 3620 can correspond to a group of servers, and each of these servers can share a database or can have their own database. The databases 3615 and 3625 can warehouse (e.g. store) information such as algorithms for deriving phototherapy parameters for specific dosages and specific phototherapy systems, patient information, and/or other information necessary for the implementation of the systems and methods described above with respect to Figures 1-34. Though the databases 3615 and 3625 are displayed logically as single units, the databases 3615 and 3625 can each be a distributed computing environment encompassing multiple computing devices, can be located within their corresponding server, or can be located at the same or at geographically disparate physical locations.

The network 3630 can be a local area network (LAN) or a wide area network (WAN), but can also be other wired or wireless networks. The network 3630 may be the Internet or some other public or private network. The client computing devices 3605 can be connected to the network 3630 through a network interface, such as by wired or wireless communication. While the connections between the server 3610 and servers 3620 are shown as separate connections, these connections can be any kind of local, wide area, wired, or wireless network, including the network 3630 or a separate public or private network.

### VIII. Phototherapy for Dermatological Disorders

Ultraviolet phototherapy has been used for several years as a treatment for dermatological disorders because of the immune modulating response from the skin. Due to its anti-proliferative and pro-differentiating effects, oral and topical calcitriol is also administered for the treatment of dermatological disorders. Phototherapy is often combined with topical calcitriol to improve efficacy and reduce total UV exposure. Low serum 25-hydroxyvitamin D₃ is correlated to dermatological disorders, so increased blood concentration from UVB phototherapy may also contribute to efficacy. UV exposure-mediated immune response, calcitriol production, vitamin D₃ production, and erythema are all highly wavelength dependent. In various embodiments of the present technology, dosage for UV phototherapy is based on minimal erythemal dose (MED), which is dictated by the erythema action spectrum. Isolating and delivering to the skin a small wavelength range of UV radiation focused between about 298 nm and 307 nm, while minimizing or eliminating UV radiation outside this target range is expected to maximize phototherapy efficacy for dermatological disorders while reducing or minimizing total UV exposure.

There are numerous advantages associated with this new technology of isolating and delivering UV radiation focused around 302 nm for phototherapy. For example, phototherapy treatments using the dosages and parameters outlined above can enhance the efficacy of the treatment of the dermatological disorder (e.g., psoriasis), while also minimizing the exposure time and total UV exposure per phototherapy treatment based on the erythema action spectrum. The dosages and parameters can also be used to decrease or minimize the UV exposure per phototherapy treatment to achieve immune suppression and biological response based on several immune response action spectra.

In addition, the dosages and parameters can provide phototherapy treatments with the least number of phototherapy sessions to treat dermatological disorders based on the psoriasis clearance action spectrum and several immune response action spectra, the cutaneous production of vitamin D₃ and the resultant correction of 25-hydroxyvitamin D₃ insufficiency, the cutaneous production of vitamin D₃ and the resultant regulation of cathelicidin expression, and the calcitriol action spectrum and resultant epidermal production of calcitriol. Moreover, the dosages and parameters can provide phototherapy treatment with reduced or minimized levels of UV exposure per phototherapy treatment session needed to achieve maximum cutaneous calcitriol production. Thus, the disclosed phototherapy devices and methods provide the efficacy equivalent of UV stimulated immune response plus oral or topical calcitriol administration using UV phototherapy stimulating cutaneous calcitriol production, and thereby provide an endogenous alternative for oral or topical calcitriol administration for the treatment of dermatological disorders.

### IX. Phototherapy for Other Indications

Phototherapy treatment methods and systems configured in accordance with the present technology can also be used to treat other diseases or indications that are affected by systematic immune abnormalities. For example, autoimmune disorders, such as multiple sclerosis ("MS"), result from overactive immune responses, and are therefore expected to benefit from the systemic immune suppressing effect of UV light exposure. An immune system response phototherapy action spectrum (e.g., similar to that shown in Figure 8) can be created by combining the contact sensitivity action spectrum of Figure 3, the cis-urocanic acid action spectrum of Figure 4, the in vivo thymine diner action spectrum of Figure 5, the in vivo tumor necrosis factor alpha action spectrum of Figure 6, and/or action spectra of peptides, hormones, and/or other biological factors related to immune suppression. These additional action spectra can include one or more action spectra of biological factors associated with the specific autoimmune disease sought to be treated. In certain embodiments, the combined immune response phototherapy action spectrum can include or be combined with the vitamin D₃/calcitriol action spectrum of Figure 10 to create a combination phototherapy action spectrum for treating one or more autoimmune diseases. This action spectrum (either including or omitting the vitamin D₃/calcitriol action spectrum) can be used to determine the wavelengths that provide the maximum phototherapy efficiency for the specific autoimmune diseases. For example, in numerous embodiments it is expected that the wavelengths with the maximum efficacy for immune response will be between 298 nm to 307 nm (e.g., focused around 302 nm). This wavelength information can then be used to select or design a phototherapy system with one or more UV sources that emit UV radiation within the desired spectrum. The parameters of each phototherapy session can then be determined based on the desired MED (e.g., 0.2 MED to 0.9 MED), the intensity of the UV source, the percentage of skin exposure, and the patient's skin type. For example, the time of each phototherapy session for a UV source with a given spectrum irradiance and intensity can be determined based on the desired MED (e.g., 0.2 MED to 0.9 MED) and the patient's skin type.

### X. Examples

The following Examples are illustrative of several embodiments of the present technology.
1. A phototherapeutic system for treating dermatological disorders, the phototherapeutic system comprising:
   a radiation source configured to emit light and having an intensity, wherein at least 75% of the light emitted by the radiation source has wavelength with a bandwidth between 298 nm and 307 nm; and
   a controller operably connected to the radiation source and configured to determine a dosage for a phototherapy session, wherein the dosage is equivalent to a product of the intensity of the radiation source and an exposure time of the radiation source, wherein the dosage has an upper bound less than 1 minimal erythema dose (MED), and wherein delivery of the dosage stimulates production of calcitriol and vitamin D₃ and provides an immune response to treat the dermatological disorder.
2. The phototherapeutic system of clam 1 wherein the radiation source is configured to expose at least 30% of a patient's skin to the light emitted by the radiation source.
3. The phototherapeutic system of example 1 wherein the radiation source is a low-energy radiation source and is configured to be positioned within 3 cm of a treatment area.
4. The phototherapeutic system of example 3 wherein the radiation source comprises an array of LEDs.
5. The phototherapeutic device of example 1, further comprising:
   a wearable substrate, and
   wherein the radiation source comprises a plurality of LEDs arranged on the wearable substrate and configured to emit light within a treatment area.
6. The phototherapeutic device of example 5 wherein the LEDs are configured to emit a substantially uniform UV radiation across the treatment area.
7. The phototherapeutic device of any one of examples 1-6, further comprising a sensor on the wearable substrate, wherein the sensor is configured to determine proximity of the radiation sources to a patient's skin.
8. The phototherapeutic device of any one of examples 1-6, further comprising a sensor configured to measure skin absorption and/or reflection, wherein the controller is configured to select dosage based on the skin absorption and/or reflection measured by the sensor.
9. The phototherapeutic device of example 1 wherein the radiation source comprises a plurality of high-energy radiation sources configured to emit light of substantially equal intensity to the treatment area.
10. The phototherapeutic device of example 9 wherein the plurality of high-energy radiation sources are configured to be spaced apart from the treatment area by about 10-100 cm, and wherein variations in distances between the high-energy radiation sources and the treatment area are less than 30 cm.
11. The phototherapeutic system of example 1 wherein the radiation source comprises at least one narrow-band UVB source.
12. The phototherapeutic system of example 1 wherein the radiation source comprises at least one broad-band UVB source.
13. The phototherapeutic system of any one of examples 1-12 wherein the intensity of the radiation source is an erythemally weighted irradiance equal to a summation of the product of an absolute measured intensity for each wavelength of light emitted by the radiation source and an erythema reference action spectrum weighting factor.
14. A method of treating dermatological disorders with a phototherapy system, the method comprising:
   determining a skin type of a user;
   determining, via a controller, a dosage of phototherapy to deliver to the user during a phototherapy session, wherein the dosage is equivalent to a product of the intensity of a radiation source of the phototherapy device and an exposure time of the radiation source, and wherein the dosage has an upper bound less than 1 minimal erythema dose (MED); and
   delivering the dose of phototherapy to a treatment area on the user via the phototherapy device, wherein delivering the dose of phototherapy comprises emitting light from the radiation source having one or more wavelengths within a bandwidth of 298-307 nm, wherein delivery of the dose of phototherapy stimulates production of calcitriol and vitamin D₃ and provides an immune response to treat the dermatological disorder.
15. The method of example 14 wherein determining the skin type of the user comprises measuring, via a sensor, skin reflectance or absorption of the user.
16. The method of example 14 or 15, further comprising determining the intensity of the radiation source by summing the product of an absolute measured intensity for each wavelength of light emitted by the radiation source and an erythema reference action spectrum weighting factor.
17. The method of any one of examples 14-16 wherein:
   delivering the dose of phototherapy comprises emitting light from a plurality of high-energy radiation sources; and
   the method further comprises positioning the treatment area of the user apart from the radiation sources by less than 100 cm, wherein variations in distance between the high-energy radiation sources and the treatment area are less than 30 cm.
18. The method of any one of examples 14-17 wherein delivering the dose of phototherapy comprises delivering the dose of phototherapy to at least 30% of the user's skin.
19. The method of any one of examples 14-16 wherein:
   delivering the dose of phototherapy comprises emitting light from a plurality of low-energy radiation sources arranged on a wearable substrate; and
   the method further comprises positioning the treatment area of the user apart from the low-intensity radiation sources by less than 3 cm and maintaining a substantially uniform distance between the treatment area and the radiation sources during the exposure time.
20. The method of any one of examples 14-19, further comprising adjusting, via the controller, exposure time and intensity of the radiation source in relation to each other to select the dosage.
21. A phototherapy system configured to stimulate vitamin D production in the skin,
   a plurality of radiation sources configured to emit light having a wavelength with a bandwidth between 298 nm and 307 nm, wherein the plurality of radiation sources are configured to emit a substantially uniform intensity of emissions across a treatment area, wherein the treatment area is a portion of a patient's skin irradiated by the radiation sources; and
   a controller operably connected to the radiation source and configured to determine a dosage for a phototherapy session, wherein the dosage is equivalent to a product of the intensity of the radiation source and an exposure time of the radiation source, wherein the dosage has an upper bound less than 1 minimal erythema dose (MED), and wherein delivery of the dosage stimulates production of calcitriol and vitamin D₃.

### Conclusion

From the foregoing, it will be appreciated that specific embodiments of the technology have been described herein for purposes of illustration, but that various modifications may be made without deviating from the disclosure. Certain aspects of the new technology described in the context of particular embodiments may be combined or eliminated in other embodiments. Additionally, although advantages associated with certain embodiments of the new technology have been described in the context of those embodiments, other embodiments may also exhibit such advantages and not all embodiments need necessarily exhibit such advantages to fall within the scope of the technology. Accordingly, the disclosure and associated technology can encompass other embodiments not expressly shown or described herein.

## Claims

1. A phototherapeutic system for treating dermatological disorders, the phototherapeutic system comprising:
a wearable substrate;
a radiation source comprising an array of LEDs arranged on the wearable substrate and configured to emit light within a treatment area, wherein at least 75% of the light emitted by the radiation source has wavelength with a bandwidth between 298 nm and 307 nm;
a controller operably connected to the radiation source and configured to determine a dosage for a phototherapy session, wherein the dosage is equivalent to a product of an intensity of the radiation source and an exposure time of the radiation source, wherein the dosage has an upper bound less than 1 minimal erythema dose (MED), and wherein delivery of the dosage to the treatment area via the radiation source stimulates production of calcitriol and vitamin D₃ and provides an immune response to treat the dermatological disorder; and
a sensor on the wearable substrate, the sensor being configured to determine proximity of the radiation source to a patient's skin.

2. The phototherapeutic system of clam 1, wherein the radiation source is configured to expose at least 30% of the patient's skin to the light emitted by the radiation source.

3. The phototherapeutic system of claim 1 or 2, wherein the radiation source is a low-energy radiation source and is configured to be positioned within 3 cm of the treatment area.

4. The phototherapeutic device of any one of claims 1-3, wherein the LEDs are configured to emit a substantially uniform UV radiation across the treatment area.

5. The phototherapeutic device of any one of claims 1-4, further comprising a sensor configured to measure skin absorption and/or reflection, wherein the controller is configured to select dosage based on the skin absorption and/or reflection measured by the sensor.

6. The phototherapeutic system of claim 1, wherein the radiation source comprises at least one narrow-band UVB source.

7. The phototherapeutic system of any one of claims 1-6, wherein the intensity of the radiation source is an erythemally weighted irradiance equal to a summation of the product of an absolute measured intensity for each wavelength of light emitted by the radiation source and an erythema reference action spectrum weighting factor.

8. The phototherapeutic system of any one of claims 1-7, wherein the controller is configured to determine the intensity of the radiation source by summing the product of an absolute measured intensity for each wavelength of light emitted by the radiation source and an erythema reference action spectrum weighting factor.

9. The phototherapeutic system of any one of claims 1-8, further comprising a plurality of lenses over the individual LEDs and disposed between the LEDs and the treatment area, wherein the plurality of lenses are configured to spread light emitted by the LEDs at least substantially evenly across a surface area.

10. The phototherapeutic system of any one of claims 1-8, wherein the plurality of LEDs are arranged in at least one array, and wherein the phototherapeutic system further comprises a lens over the array and disposed between the LEDs and the treatment area, wherein the lens is configured to spread light emitted by the LEDs of the array at least substantially evenly across a surface area.

## Patentansprüche

1. Lichttherapiesystem zur Behandlung dermatologischer Erkrankungen, wobei das Lichttherapiesystem umfasst:
ein am Körper tragbares Substrat;
eine Strahlungsquelle umfassend ein Array von LEDs, die auf dem am Körper tragbaren Substrat angeordnet und konfiguriert sind, Licht innerhalb eines Behandlungsbereichs auszustrahlen, wobei mindestens 75 % des von der Strahlungsquelle ausgestrahlten Lichts eine Wellenlänge mit einer Bandbreite zwischen 298 nm und 307 nm aufweisen;
ein Steuergerät, das in Wirkverbindung mit der Strahlungsquelle und konfiguriert ist, eine Dosierung für eine Lichttherapiesitzung zu bestimmen, wobei die Dosierung gleichbedeutend mit einem Produkt einer Intensität der Strahlungsquelle und einer Einwirkzeit der Strahlungsquelle ist, wobei die Dosierung eine Obergrenze aufweist, die weniger als 1 minimale Erythemdosis (MED) ist, und wobei die Abgabe der Dosierung an den Behandlungsbereich über die Strahlungsquelle die Produktion von Calcitriol und Vitamin D₃ stimuliert und für eine Immunreaktion zur Behandlung der dermatologischen Erkrankung sorgt; und
einen Sensor auf dem am Körper tragbaren Substrat, wobei der Sensor konfiguriert ist, die Nähe der Strahlungsquelle zur Haut eines Patienten zu bestimmen.

2. Lichttherapiesystem nach Anspruch 1, wobei die Strahlungsquelle konfiguriert ist, mindestens 30 % der Haut des Patienten dem von der Strahlungsquelle ausgestrahlten Licht auszusetzen.

3. Lichttherapiesystem nach Anspruch 1 oder 2, wobei die Strahlungsquelle eine energiearme Strahlungsquelle ist und konfiguriert ist, innerhalb von 3 cm des Behandlungsbereichs positioniert zu sein.

4. Lichttherapievorrichtung nach einem der Ansprüche 1-3, wobei die LEDs konfiguriert sind, eine im Wesentlichen einheitliche UV-Strahlung über den Behandlungsbereich auszustrahlen.

5. Lichttherapievorrichtung nach einem der Ansprüche 1-4, ferner umfassend einen Sensor, der konfiguriert ist, Hautabsorption und/oder -reflektion zu messen, wobei das Steuergerät konfiguriert ist, eine Dosierung auf Basis der von dem Sensor gemessenen Hautabsorption und/oder -reflektion auszuwählen.

6. Lichttherapiesystem nach Anspruch 1, wobei die Strahlungsquelle mindestens eine schmalbandige UVB-Quelle umfasst.

7. Lichttherapiesystem nach einem der Ansprüche 1-6, wobei die Intensität der Strahlungsquelle eine erythem gewichtete Irradianz gleich einer Summierung des Produkts einer absoluten gemessenen Intensität für jede von der Strahlungsquelle ausgestrahlte Lichtwellenlänge und eines Erythem-Referenzwirkungsspektrum-Gewichtungsfaktors ist.

8. Lichttherapiesystem nach einem der Ansprüche 1-7, wobei das Steuergerät konfiguriert ist, die Intensität der Strahlungsquelle durch Summieren des Produkts einer absoluten gemessenen Intensität für jede von der Strahlungsquelle ausgestrahlte Lichtwellenlänge und eines Erythem-Referenzwirkungsspektrum-Gewichtungsfaktors zu bestimmen.

9. Lichttherapiesystem nach einem der Ansprüche 1-8, ferner umfassend eine Mehrzahl von Linsen über den individuellen LEDs und mit Anordnung zwischen den LEDs und dem Behandlungsbereich, wobei die Mehrzahl von Linsen konfiguriert ist, von den LEDs ausgestrahltes Licht mindestens im Wesentlichen gleichmäßig über eine Oberfläche zu verbreiten.

10. Lichttherapiesystem nach einem der Ansprüche 1-8, wobei die Mehrzahl von LEDs in mindestens einem Array angeordnet ist und wobei das Lichttherapiesystem ferner eine Linse über dem Array und mit Anordnung zwischen den LEDs und dem Behandlungsbereich umfasst, wobei die Linse konfiguriert ist, von den LEDs des Arrays ausgestrahltes Licht mindestens im Wesentlichen gleichmäßig über eine Oberfläche zu verbreiten.

## Revendications

1. Système photothérapeutique pour traiter des troubles dermatologiques, le système photothérapeutique comprenant :
un substrat portable ;
une source de rayonnement comprenant un réseau de LED agencées sur le substrat portable et configurées pour émettre de la lumière dans une zone de traitement, au moins 75 % de la lumière émise par la source de rayonnement ayant une longueur d'onde avec une bande passante comprise entre 298 nm et 307 nm ;
un contrôleur connecté de manière fonctionnelle à la source de rayonnement et configuré pour déterminer une dose pour une séance de photothérapie, la dose étant équivalente à un produit d'une intensité de la source de rayonnement et d'un temps d'exposition de la source d'exposition, la dose ayant une limite haute inférieure à 1 érythème perceptible minimum (MED) et l'administration de la dose à la zone de traitement par l'intermédiaire de la source de rayonnement stimulant la production de calcitriol et de vitamine D₃ et fournissant une réponse immunitaire pour traiter le trouble dermatologique ; et
un capteur sur le substrat portable, le capteur étant configuré pour déterminer une proximité de la source de rayonnement à la peau d'un patient.

2. Système photothérapeutique selon la revendication 1, dans lequel la source de rayonnement est configurée pour exposer au moins 30 % de la peau du patient à la lumière émise par la source de rayonnement.

3. Système photothérapeutique selon la revendication 1 ou 2, dans lequel la source de rayonnement est une source de rayonnement à faible énergie et est configurée pour être positionnée à 3 cm ou moins de la zone de traitement.

4. Système photothérapeutique selon l'une quelconque des revendications 1 à 3, dans lequel les LED sont configurées pour émettre un rayonnement UV sensiblement uniforme sur toute la zone de traitement.

5. Système photothérapeutique selon l'une quelconque des revendications 1 à 4, comprenant en outre un capteur configuré pour mesurer une absorption cutanée et/ou une réflexion cutanée, le contrôleur étant configuré pour sélectionner une dose sur la base de l'absorption cutanée et/ou de la réflexion cutanée mesurées par le capteur.

6. Système photothérapeutique selon la revendication 1, dans lequel la source de rayonnement comprend au moins une source d'UVB à bande étroite.

7. Système photothérapeutique selon l'une quelconque des revendications 1 à 6, dans lequel l'intensité de la source de rayonnement est une irradiation pondérée au niveau de l'érythème égale à une addition du produit d'une intensité absolue mesurée pour chaque longueur d'onde de lumière émise par la source de rayonnement et d'un facteur de pondération du spectre d'action de référence de l'érythème.

8. Système photothérapeutique selon l'une quelconque des revendications 1 à 7, dans lequel le contrôleur est configuré pour déterminer l'intensité de la source de rayonnement en additionnant le produit d'une intensité absolue mesurée pour chaque longueur d'onde de lumière émise par la source de rayonnement et d'un facteur de pondération du spectre d'action de référence de l'érythème.

9. Système photothérapeutique selon l'une quelconque des revendications 1 à 8, comprenant en outre, sur les LED individuelles, une pluralité de lentilles qui sont disposées entre les LED et la zone de traitement, la pluralité de lentilles étant configurées pour diffuser la lumière émise par les LED de façon au moins sensiblement homogène sur toute une superficie.

10. Système photothérapeutique selon l'une quelconque des revendications 1 à 8, dans lequel la pluralité de LED sont agencées en au moins un réseau, et le système photothérapeutique comprenant en outre, sur le réseau, une lentille qui est disposée entre les LED et la zone de traitement, la lentille étant configurée pour diffuser la lumière émise par les LED du réseau de façon au moins sensiblement homogène sur toute une superficie.
